(19) Europäisches Patentamt / European Patent Office / Office européen des brevets

(11) **EP 2 277 047 B1**

(12) **EUROPEAN PATENT SPECIFICATION**

(45) Date of publication and mention
of the grant of the patent:
**05.11.2014   Bulletin 2014/45**

(51) Int Cl.:
**G01N 33/542** *(2006.01)*     **G01N 33/569** *(2006.01)*
**G01N 33/58** *(2006.01)*

(21) Application number: **09725807.3**

(22) Date of filing: **27.03.2009**

(86) International application number:
**PCT/GB2009/050303**

(87) International publication number:
**WO 2009/118570 (01.10.2009 Gazette 2009/40)**

(54) **Time-resolved fluorescence energy transfer (TR-FRET) assay for detecting exposure to an infectious organism**

Zeitaufgelöster Fluoreszenzenergietransfer-(TR-FRET-)test zum Nachweis eines Inkontaktkommens mit einem infektiösen Organismus

Procédé de détection de l'exposition à un organisme infectieux par transfert d'énergie par résonance de type Förster en temps résolu (TR-FRET)

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority:  **28.03.2008   GB 0805608**

(43) Date of publication of application:
**26.01.2011   Bulletin 2011/04**

(60) Divisional application:
**13161174.1 / 2 618 148**

(73) Proprietor: **The Secretary of State for Environment,
Food and Rural Affairs
Worcester WR5 2LQ (GB)**

(72) Inventor: **MCGIVEN, John
Surrey KT15 3NB (GB)**

(74) Representative: **Turner, Rhiannon Rosalind et al
Greaves Brewster LLP
Copa House
Station Road
Cheddar, BS27 3AH (GB)**

(56) References cited:
**WO-A1-2005/000883     WO-A2-2004/041865
WO-A2-2008/048970     US-A1- 2006 234 215
US-A1- 2006 240 571**

- **QIN QIU-PING ET AL: "Time-resolved fluorescence resonance energy transfer assay for point-of-care testing of urinary albumin."** CLINICAL CHEMISTRY JUL 2003, [Online] vol. 49, no. 7, July 2003 (2003-07), pages 1105-1113, XP002538503 ISSN: 0009-9147 Retrieved from the Internet: URL:http://www.clinchem.org/cgi/reprint/49 /7/1105> [retrieved on 2009-07-22]
- **GALL D ET AL: "Fluorescence polarization assay for detection of Brucella abortus antibodies in bulk tank bovine milk samples."** CLINICAL AND DIAGNOSTIC LABORATORY IMMUNOLOGY NOV 2002, [Online] vol. 9, no. 6, November 2002 (2002-11), pages 1356-1360, XP002538523 ISSN: 1071-412X Retrieved from the Internet: URL:http://cvi.asm.org/cgi/reprint/9/6/135 6> [retrieved on 2009-07-22]
- **GARIN-BASTUJI B ET AL: "The diagnosis of brucellosis in sheep and goats, old and new tools"** SMALL RUMINANT RESEARCH, ELSEVIER, AMSTERDAM, NL, vol. 62, no. 1-2, 1 March 2006 (2006-03-01), pages 63-70, XP025117948 ISSN: 0921-4488 [retrieved on 2006-03-01]

- NEWBY D T ET AL: "REAL-TIME PCR DETECTION OF BRUCELLA ABORTUS: A COMPARATIVE STUDY OF SYBR GREEN I, 5'-EXONUCLEASE, AND HYBRIDIZATION PROBE ASSAYS" APPLIED AND ENVIRONMENTAL MICROBIOLOGY, AMERICAN SOCIETY FOR MICROBIOLOGY, US, vol. 69, no. 8, 1 August 2003 (2003-08-01), pages 4753-4759, XP008070970 ISSN: 0099-2240
- ANONYMOUS: "Atlas Brucella Test (Rose Bengal)" ATLAS MEDICAL, [Online] 12 June 2004 (2004-06-12), XP002538539 Retrieved from the Internet: URL:http://www.atlas-site.co.uk/index_ file s/website/ $8.01.00.0.0050%20Brucella%20Ros e%20Bengal%2050%20T%20S06.pdf> [retrieved on 2009-07-22]
- HINCHLIFFE P M ET AL: "Detection of complement fixation by enzyme linked immunosorbant assay (COMPELISA)." JOURNAL OF CLINICAL PATHOLOGY JAN 1983, [Online] vol. 36, no. 1, January 1983 (1983-01), pages 100-103, XP002538540 ISSN: 0021-9746 Retrieved from the Internet: URL:http: //jcp.bmj.com/cgi/reprint/36/1/10 0> [retrieved on 2009-07-22]
- BORAKER D K ET AL: "BrucELISA: an enzyme-antibody immunoassay for detection of Brucella abortus antibodies in milk: correlation with the Brucella ring test and with shedding of viable organisms." JOURNAL OF CLINICAL MICROBIOLOGY OCT 1981, [Online] vol. 14, no. 4, October 1981 (1981-10), pages 396-403, XP002538541 ISSN: 0095-1137 Retrieved from the Internet: URL:http: //www.pubmedcentral.nih.gov/picre nder.fcgi? artid=271991&blobtype=pdf> [retrieved on 2009-07-22]
- NEWBY D T ET AL: "REAL-TIME PCR DETECTION OF BRUCELLA ABORTUS: A COMPARATIVE STUDY OF SYBR GREEN I, 5'-EXONUCLEASE, AND HYBRIDIZATION PROBE ASSAYS", APPLIED AND ENVIRONMENTAL MICROBIOLOGY, AMERICAN SOCIETY FOR MICROBIOLOGY, US, vol. 69, no. 8, 1 August 2003 (2003-08-01) , pages 4753-4759, XP008070970, ISSN: 0099-2240, DOI: 10.1128/AEM. 69.8.4753-4759.2003
- BINZ H K ET AL: "Engineered proteins as specific binding reagents", CURRENT OPINION IN BIOTECHNOLOGY, LONDON, GB, vol. 16, no. 4, 1 August 2005 (2005-08-01) , pages 459-469, XP027676733, ISSN: 0958-1669 [retrieved on 2005-08-01]
- NORD K ET AL: "Binding proteins selected from combinatorial libraries af an alpha-helical bacterial receptor domain", NATURE BIOTECHNOLOGY, NATURE PUBLISHING GROUP, NEW YORK, NY, US, vol. 15, no. 8, 1 August 1997 (1997-08-01) , pages 772-777, XP002149252, ISSN: 1087-0156
- SILVERMAN JOSHUA ET AL: "Multivalent avimer proteins evolved by exon shuffling of a family of human receptor domains", NATURE BIOTECHNOLOGY, NATURE PUBLISHING GROUP, NEW YORK, NY, US, vol. 23, no. 12, 20 November 2005 (2005-11-20), pages 1556-1561, XP009088629, ISSN: 1087-0156, DOI: 10.1038/NBT1166

**Description**

**Field of invention**

The present invention relates to a method for the detection of diagnostic moieties, in particular to methods based upon Time Resolved Fluorescent Energy Transfer (TR-FRET) technology to measure the proximity of moieties such as antibodies or antigens in biological samples, which is useful in the diagnosis and screening for diseases caused by infectious organisms such as brucellosis.

**Background**

FRET technology has been known for many years. In FRET, a donor fluorophore is excited by light, and if a suitable acceptor is in close proximity, the excited state energy from the donor can be transferred to the acceptor. For the acceptor to be suitable it must have an excitation wavelength that overlaps with the emission wavelength of the donor. The energy transfer leads to a decrease in the donor's emission intensity and an increase in the acceptor's emission intensity. If the two fluorophores emit light at different wavelengths then spectral filtration allows measurement of their individual intensities. The degree to which the energy transfer occurs depends on the inverse sixth-distance between donor and acceptor. Thus, the relative intensities of the fluorophores provides a measurement of the distance between the two.

Time resolved FRET (TR-FRET) (Morrison, L.E., 1988. Anal. Biochem., 174 (1) 101) adds another dimension to the technique. TR-FRET was considerably improved by the development of rare earth lanthanide chelates to act as donor fluorophores in the TR-FRET reaction. This improvement was due to the long fluorescent lifetimes of these donors which allowed for longer time gating periods, thus eliminating more non-specific fluorescence. Lanthanide chelate labels such as terbium are used in this application as they have long fluorescent lifetimes. Natural fluorescence of organic components after light excitation has taken place will produce a background reading. However the fluorescence lifetime of terbium far exceeds that of the background noise. By delaying the time between light emission and measurement (gating), this background can be eliminated from the assay. As a result of temporal filtration the sensitivity of the assay can be improved.

Suitable lanthanide chelates useful in the method include those described for example in US Patent Nos 5,622,821, 5,639,615, 5,656,433 and 4,822,733.

TR-FRET is a widely utilised technique in the pharmaceutical industry for compound analysis and drug discovery. In these circumstances, it is applied to relatively pure samples of compounds which are laboratory derived. It may be used in high-throughput screening to screen large numbers of compounds for their ability to interact with a particular biological moiety such as a receptor.

The technique has not previously been applied to biological samples for the detection of diagnostic moieties for infectious diseases. Generally such methods are carried out on samples such as blood, serum, milk, urine or cerebrospinal fluid samples which, in contrast to the samples used in drug screening, are highly heterogeneous samples, and may contain fluorescence inhibitors. Previous attempts to increase the sensitivity have focused on the addition of additional reagents such as fluoride ions (see US Patent No. 5,627,074) but this has the effect of further complicating the assay, and the results have not been sufficient to ensure that the technique has found widespread use in diagnosis.

US2006/0240571 discusses the potential of using a FRET-based system for detection of chemicals and microorganisms in foodstuffs. However, the only data provided is for *E. coli* in known dilutions in phosphate buffered saline, also a non-heterogeneous sample.

Furthermore, diagnosis of disease is relatively infrequently carried out on the basis of high throughput screening.

Brucellosis is a zoonotic disease of global significance. The disease is caused by bacteria of the genus *Brucella* which themselves belong to the $\alpha$-2 subdivision of Proteobacteria. The genus consists of six classical species, *B.abortus, B.melitensis, B.suis, B.ovis, B.canis,* and *B.neotomae* plus more recently discovered strains from marine mammals. Of the *Brucella* species, *B.abortus, B.melitensis* and *B.suis* are of principal human health and economic importance. These species have smooth lipopolysaccharide (LPS) which is considered a major virulence factor of disease (Porte, et al 2003. Infect. Immunol., 71 (3) 1481) whereas *B.ovis* and *B.canis* have rough LPS.

Brucellosis is widespread and has only been eradicated from a small number of countries, including Great Britain. Even here its re-introduction remains a real threat to livestock and human health as well as the rural economy. As such the detection of *Brucella* in livestock is a major issue facing any country with a livestock industry. In order to qualify for OIE (Office International des Epizooties) disease free status, a country must have ceased vaccination for at least three years. The disease must then be controlled by serological testing, conducted periodically in each herd (OIE Terrestrial Animal Health Code 2007). Once the country has been declared disease free, presumptive diagnosis based on serological testing must continue for five years whereupon the system for control can be decided locally. In the few countries to have eradicated the disease, maintenance of 'OIE disease free' status requires considerable investment in surveillance strategies.

The economic burden of effective brucellosis surveillance, where large numbers of serum and/or milk samples are surveyed annually is high.

The OIE prescribed an alternative serological tests (Nielsen, K., Ewalt, D.R., 2004. Bovine brucellosis. Manual of standards for diagnostic tests and vaccines. Office International Des Epizooties, Paris, 409-38) for brucellosis due to infection with smooth strains rely largely upon the measurement of the host's generated antibody response to the O-antigen of the smooth LPS. Classical tests include the Rose Bengal Test (RBT), the Complement Fixation Test (CFT) and the Serum Agglutination Test (SAT) all of which employ a whole cell antigen as the key diagnostic reagent. More contemporary techniques such as the indirect (i) ELISA, competitive (c) ELISA and the Fluorescent Polarisation Assay (FPA) employ purified LPS or O-antigen as the diagnostic reagent. The immunodominance of the LPS O-antigen is the basis for the generally good sensitivity of these assays.

High throughput serological testing is an essential element in monitoring brucellosis and the ELISA tests are the most readily amenable to this due to the standardised nature of the technology and reagents. This allows for many efficiency savings including the introduction of automation. Despite the advantages of ELISA over the more traditional tests in this regard, the ELISA still requires several steps to complete including separation steps. Although these steps can be automated they are a vital part of the assay and are a frequent source of imprecision, error and mechanical breakdown.

Assays which have the advantages of the ELISA, such as a 96 well format, objective assessment and good sensitivity and specificity parameters, but which reduce the burden of work and opportunity for error are desirable.

The Fluorescent Polarisation Assay (FPA) for the detection of antibodies to *Brucella* OPS (O-antigen of Lipopolysaccharide) (Neilsen at al. Journal of Immunological Methods (1996) 195, Issues 1-2, p161-168) is a rapid homogeneous test. However, there are a number of drawbacks. Each sample must be read twice, once before the diagnostic antigen is added, and once after. The results can be significantly affected by relatively small changes in ambient temperature of just a few degrees centigrade (Minas et al., Journal of Immunological Methods (2007) 320, 1-2, p94-103) which negatively effects the reproducibility of the assay. The test also requires the use of a highly purified antigen which increases production costs which are in turn passed on to the customer.

US2006/234215 discloses a bioswitch which includes a long-lived emitter such as a lanthanide luminophore for time gated detection of ligand binding.

Qin et al, (2003; Clin Chem: vol 49: 1105-1113) discusses a TR-FRET essay for testing of urinary albumin.

## Summary of Invention

According to the present invention there is provided a method for detecting the presence of a diagnostic moiety, indicative of exposure of a human or animal to a *Brucella* species infectious organism, in a biological sample taken from the human or animal, said method comprising;

(a) adding to said sample a first fluorescently labelled reagent which binds said diagnostic moiety and a second fluorescently labelled reagent which either binds said diagnostic moiety in addition to said first fluorescently labelled reagent, or which binds the first fluorescently labelled reagent or a complex comprising the first fluorescently labelled reagent in competition to the said diagnostic moiety, wherein a label on one of the first or second fluorescently labelled reagent acts as a fluorescent energy donor compound and wherein the label on the other of the first or second fluorescently labelled reagent acts as a fluorescent energy acceptor compound which is able to accept fluorescent energy from said donor compound;

(b) exciting the fluorescent energy donor compound by illuminating with light of a wavelength which is absorbed by said fluorescent energy donor compound;

(c) measuring fluorescent signal emitted by said fluorescent energy acceptor compound as a result of its absorption of the fluorescent energy from the donor compound after a time delay but within said period of time; and

(d) relating the results to the presence or absence of diagnostic moiety in said sample;

wherein the diagnostic moiety is selected from the *Brucella* species infectious organism, an antigen of the *Brucella* species infectious organism, or an antibody to an antigen of the *Brucella* species infectious organism, and wherein the first and second fluorescently labelled reagents are specific binding reagents.

The period of time over which the signal from the donor compound is emitted may be longer than the period of time for which a signal is emitted by the acceptor compound.

The applicants have found that this technique (essentially TR-FRET), can be successfully applied to biological samples used in diagnosis of a diverse range of infectious diseases.

Using the method of the invention provides a new rapid homogenous assay for the effective detection of diagnostic moieties of infectious diseases such as brucellosis. The applicants have found that TR-FRET can be adapted for use as a diagnostic test, using reagents which are relatively easily prepared and which do not require extensive preparation. As such, it provides considerable efficiency savings as compared to a conventional ELISA protocol for instance.

Suitably, the fluorescent signal produced by the donor as well as the acceptor compound is measured in step (c). This allows the ratio of the signals to be calculated, and this provides a clearer indication of the occurrence of FRET and thus the presence or absence of diagnostic moiety in the sample. In particular, the intensity of the light emitted by both the donor and the acceptor are measured in step (c) and then the acceptor intensity is divided by the donor intensity to generate a TR-FRET ratio. This ratio can then be used to express the results for each sample.

The use of ratiometric calculations with the results is particularly suitable for assays on samples with variable matrix compositions (e.g. sera etc) as the ratiometric results method provides some level of resistance from the effects of fluorescence quenching caused by the sample matrix, as compared with the simple intensity results.

In order to ensure that the results of the assay are as accurate as possible, it is useful to ensure that the amount of unlabelled first and second reagent and the amount of unconjugated label (unconjugated fluorophores) is kept to a minimum. This can be achieved, at least in relation to the direct labelling of unlabelled first and second reagents, by ensuring that they are prepared using an excess of label during the conjugation procedure. However, it is then important to ensure that any excess unbound label or fluorophore is removed after the conjugation process. If the reagents are to be labelled indirectly, though the use of fluorescently labelled secondary reagents, then both the primary and secondary reagents must be titrated against each other to identify the optimal concentrations for use in the application.

In a preferred embodiment, the first fluorescently labelled reagent is labelled directly and is substantially free of any unconjugated label which acts as a fluorophore, and similarly the second fluorescently labelled reagent is labelled directly and substantially free of unconjugated label.

As used herein, the expression "substantially free" means that steps have been taken to remove unconjugated labels or fluorophores from the first and second labelled reagents which are fluorophore conjugated diagnostic reagents. In practice, this will generally mean that, after labelling, the reagent is passed down a desalting column, for example a desalting resin column such as a Zeba™ column available from Pierce, to ensure that the amount of unconjugated label is minimised.

In an embodiment, for the first and second labelled reagents, less than 10% of the corresponding fluorophores within the preparation are unconjugated, for example less than 5% and in particular less than 2%

The applicants have found that a labelling process in which a reagent is incubated for a suitable period of time with an excess of labelling reagent such as fluorescein and immediately passed down a desalting column, without any previous dialysis, provides a particularly useful method for preparing labelled reagents for use in the method of the invention. Apart from this constraint, the purity of the reagents need not be that high, since the specificity of the TR-FRET procedure will mean that any contaminants, even if labelled, will not generate significant fluorescent signals.

Therefore, the first and second reagents used for the preparation of the labelled first and second reagents respectively do not themselves have to be subjected to extensive purification procedures. The applicants have found that even relatively impure reagents can be used and the assay is able to produce meaningful results. Purification of reagents such as diagnostic antigens in particular, from all the other material that may be in a bacterial/viral/cell culture preparation can be very difficult. Therefore, this finding provides a significant advantage for the assay described herein, in that the reagent preparation may be simplified and the cost of the reagents may be kept low.

In a particular embodiment, the method is carried out as a "competition" type assay, wherein the second fluorescently labelled reagent binds the first fluorescently labelled reagent in competition to the said diagnostic moiety, and wherein a reduction of the fluorescent signal from the acceptor fluorophore (or a decrease in the ratio of the acceptor: donor signal intensity where the donor signal is also measured) measured in step (c) is indicative of the presence of diagnostic moiety in the sample. In this case, when the sample contains the diagnostic moiety, this competes with the second labelled reagent for binding to the first labelled reagent. As a result, the number of complexes formed which contain both first and second labels in relatively close proximity to each other is reduced. As a result, the signal measured in step (c) is low or absent, since relatively few acceptor compounds are in a position to be excited by the emission from the donor compound. In contrast, where the sample contains no diagnostic moiety, then the first and second labelled reagents are able to bind together. When this happens, the donor and acceptor labels are brought into close proximity to each other, so that FRET can occur between them. In the context of the present invention, the fact that the long fluorescence lifetime of the donor enables it to emit energy over a relatively long period of time that can be transferred to an acceptor, with a short fluorescence lifetime, within sufficient proximity, means that the signal from this particular interaction is longer lived than the background 'noise', and therefore a reading after a time delay, from which 'noise' is largely eliminated as defined above is possible, in accordance with normal TR-FRET procedures.

It is also possible that both the first and second labelled reagents form a complex with a substrate such as a bacterial cell, which may be formed either before addition to the assay or it may be formed in situ in the assay. In such cases, the substrate may bind both the first and second labelled binding agents to allow FRET to occur, but in the presence of the diagnostic moiety, the first or second labelled binding agent will be inhibited from binding the substrate due to competition with the diagnostic moiety. As a result, a reduction in the amount of FRET occurring will be indicative of the presence of diagnostic moiety in the sample.

However, alternative "sandwich assays" where the second fluorescently labelled reagent binds said diagnostic moiety in addition to said first fluorescently labelled reagent may be possible, in particular where the diagnostic moiety is large and can carry two labelled reagents simultaneously. In this case, where the diagnostic moiety is present in a sample, both the first and second labelled reagents are able to bind to it. This brings the first and second labels in close proximity to each other, so as to allow FRET to occur. Therefore, when carrying out a TR-FRET analysis, the presence of a significant FRET signal produced by the acceptor compound or a change in the ratio of acceptor:donor signal indicative of an increased acceptor compound signal after the time delay, will indicate the presence of the diagnostic moiety. Therefore, this information can be used in the diagnosis of the infectious disease.

The term "diagnostic moiety" means an antigen of the *Brucella* species infectious organism, or an antibody to an antigen of the *Brucella* species infectious organism, or it may comprise the *Brucella* species organism. Where the diagnostic moiety is the *Brucella* species organism, it will generally comprise multiple epitopes or other binding motifs on the surface, allowing the first and second labelled reagents to bind to different epitopes or motifs in close proximity to one another to allow FRET to occur. Where the diagnostic moiety is an antibody associated with the *Brucella* species infectious agent, particularly suitable antigens for use as labelled reagents will be immunodominant antigens, and these may include protein antigens as well as glycoconjugates such as lipopolysaccharide (LPS) antigens. Antigens associated with bacterial cell membranes may be particularly suitable in some cases.

Therefore, the method directly identifies the presence, in the sample, of a moiety as the result of exposure of a human or animal to a *Brucella* species infectious organism. There is no requirement for a general immune response to have occurred. Advantageously, this allows the user of the method to detect exposure of a human or animal to the *Brucella* species infectious organism at an early stage, even in the absence of a more general immune response. Diagnosis of infection of the human or animal by the *Brucella* species organism is enabled.

Furthermore, as mentioned above, the proximity based nature of the method allows for relatively impure preparations of antigen to be used. This may reduce the cost of antigen production techniques or enable the use of antigens whose precise identity is not known.

Antigen detection assays, where multiple identical antigen epitopes exist on a single structure, may also be developed using a single mAb which has been labelled in one instance with a lanthanide donor and in another with the appropriate acceptor. Such an assay could be developed to rapidly detect the presence of 'M dominant' *Brucella* for example using BM40 antibody.

The first and second labelled reagents are specific binding reagents. Thus the first labelled reagent will specifically bind the diagnostic moiety and the second labelled reagent will specifically bind either the diagnostic moiety or the first binding agent in competition to the diagnostic binding moiety. Specific binding pairs are well known in the art, and include antibody pairs and antibody-antigen pairs. Antibodies may be monoclonal or polyclonal, and are preferably monoclonal, but, if required, binding fragments of antibodies such as Fab, $F(ab')_2$ fragments or single chain antibody fragments may comprise the first and second labelled reagents.

Host species include mammals such as humans or animals including ruminants such as cattle and sheep as well as goats, pigs, cervids, such as deer, felines such as cats or canines such as dogs. In particular, the host are humans or livestock used in agriculture such as ruminants, pigs, chickens or other farmed fowl.

The assay is a homogeneous assay, and in accordance with the method of the invention, can be carried out simply by mixing the necessary components together in a reaction vessel, such as a well in a plate, and then subjecting them to a TR-FRET assay, as described. Equipment appropriate for this purpose is commercially available.

One of the first or second fluorescently labelled reagents is suitably a bacterial glycoconjugate such as a labelled LPS antigen of a *Brucella* species, and the other is a labelled antibody which binds said antigen.

Suitable fluorescent energy donor compounds for use in the labelled reagents of the method of the invention include lanthanide chelates as described for example in US Patent Nos 5,622,821, 5,639,615, 5,656,433 and 4,822,733, the content of which is incorporated herein by reference. In particular however, the fluorescent energy donor compound is a europium, samarium or terbium lanthanide chelate. These are known to have prolonged emission times, following excitation. The fluorescent energy acceptor compound is suitably selected to ensure that FRET occurs between the donor and the acceptor. In the case of a terbium donor, fluorescein or a derivative thereof, such as FAM, FITC, JOE etc. may be a suitable acceptor.

Where a lanthanide europium chelate is used as the donor compound, acceptor fluorophores may include Cy5, allophycocyanin (APC) and a variety of Alexa Fluor dyes, all of which emit light in the infrared spectrum. It has been suggested that emission at these wavelengths is less affected by surrounding compounds such as those found in sera and typical buffer solutions, and therefore this particular combination may be particularly advantageous in the context of the method of the present invention.

The optimal concentrations of the first and second labelled reagent added to any particular sample will vary depending upon factors such as the precise nature of the sample, the amount of diagnostic moiety likely to be found in it, the precise nature of the labels and the reagents used etc. Generally however, it may be expected that increasing the number of fluorophores per labelled reagent will increase the signal-to-noise ratio up to the point whereby the extent of the labelling restricts the binding of the reagents. These concentrations will be determined using conventional methods in accordance with standard practice, as outlined herein.

The biological samples used in the method of the invention may comprise any of the conventionally available sample types, provided any diagnostic moiety is found in them. Thus, they may include blood, serum, milk, urine, plasma, mucous, cerebrospinal fluid, faeces or tissue biopsy samples, depending upon the particular infectious organism being diagnosed. Apparatus used in the method is available commercially. These include excitation sources such as light or laser sources. Suitable light of the desired wavelengths is fed to and read from the reaction vessel using appropriate filters, as would be understood in the art.

Suitable buffers will be those that are conventional in the art. They include neutral buffers which fall within a pH range of from 6 to 8, for example at 7-7.4, such as TRIS buffered saline and phosphate buffered saline.

The time delay required to achieve a good signal from the method of the invention will depend upon various factors such as the nature of the labelled reagents, the nature of the sample, the illumination source etc. However, typically, the time delay between excitation of the donor compound and reading of the signal from the acceptor compound will be between 50 and 200 microseconds.

The applicants sought to develop a homogeneous analogue of an existing ELISA using the *Brucella* specific monoclonal antibody (mAb), BM40 - (Greiser-Wilke et al. 1985, Zentralbl Veterinarmed B. 32 (8) 616) and the *Brucella* antigen (16M LPS). These reagents are used in the *Brucella* cELISA kit developed and distributed by the VLA (UK). There are six classical species of *Brucella* (*B.abortus, B.melitensis, B.suis, B.ovis, B.canis* and *B.neotimiae*) plus some more newly discovered strains from marine mammals and small rodents. *Brucella* may have smooth or rough LPS. The most virulent stains have smooth LPS. All the reference stains for *B.abortus, B.melitensis* and *B.suis,* have smooth LPS as do the vast majority of naturally occurring field strains. These three species are the major causative agents of brucellosis and represent the biggest threat to the health of humans, bovines, caprines, ovines and porcines. The difference between rough and smooth strains is that smooth strains possess the O-antigen in addition to the core and Lipid-A. *Brucella* sLPS can be of two types, A or M dominant. This nomenclature refers to the structure of the O-antigen that contains, in addition to epitopes that are shared between the two types, each has a distinct epitope (A or M) that is not shared. In the particular embodiment of the assay described herein for the detection of *Brucella,* one of the binding reagents used is a monoclonal antibody which is anti-M and the other binding reagent is an M dominant sLPS antigen. However, the assay will still detect antibodies that have been raised against the sLPS from A dominant strains of *Brucella.* This is because each of the epitopes found on the O-antigen overlaps which leads to steric hindrance whereby an antibody against a shared sLPS epitope may displace an antibody to a non-shared epitope. This leads to competition between the antibodies which is detectable by immunoassays such as the cELISA and this TR-FRET assay. As a result, a generic diagnosis of *Brucella* infection is possible.

By labelling the antibody with terbium (donor) and the antigen with fluorescein (acceptor) any subsequent binding between antigen and antibody may bring the fluorescent probes within close enough proximity for FRET to occur. The introduction of competing antibodies or antigens may cause dissociation of the fluorescent molecules resulting in a reduction of FRET. Changes in the fluorescent signal caused by FRET may therefore indicate the presence of anti-*Brucella* antibodies in test serum.

As outlined below, a successful TR-FRET assay was developed using the *Brucella* LPS antigen and anti-LPS monoclonal antibody (BM40) currently used in the *Brucella* cELISA. This provides a simple, rapid homogenous homologue of pre-existing assays such as ELISA assays, and one that is highly amenable to automation.

Selection of suitable preparations and concentrations for the various reagents can be carried out using conventional optimisation methods, including the titration of a variety of conjugated BM40 and LPS preparations against each other. The BM40 conjugates were also titrated against equivalent concentrations of free fluorescein, simulating complete inhibition of specific FRET, so that non-specific (diffusion enhanced) FRET could be measured. These reagent combinations were judged on the ratio of specific versus non-specific FRET signals they generated, the best showing specific signals 50 times greater than the non-specific signals background when the results were expressed as a TR-FRET ratio (acceptor/donor intensities).

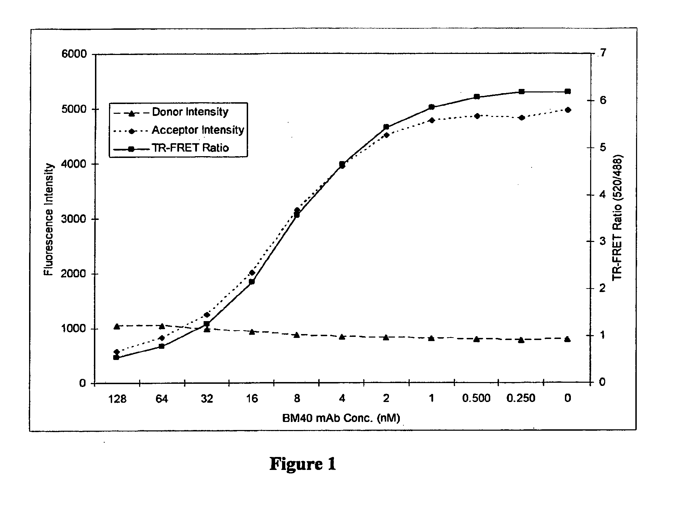

The analytical sensitivity of the TR-FRET assay was determined by adding unconjugated BM40 to the reaction and measuring the subsequent reduction in FRET due to competition for the conjugated antigen (Figure 1). The same approach was used to determine the analytical sensitivity of the cELISA.

It was found that the TR-FRET assay of the invention was twice as analytically sensitive as the cELISA. The cELISA control sera were added to the TR-FRET assay at a variety of dilutions from 1/5 to 1/80. The results demonstrated that even after only five minutes incubation, there was a clear difference between FRET signals from the Positive, Weak Positive and Negative controls. The optimal serum dilution for the assay appeared to be approximately 1/20.

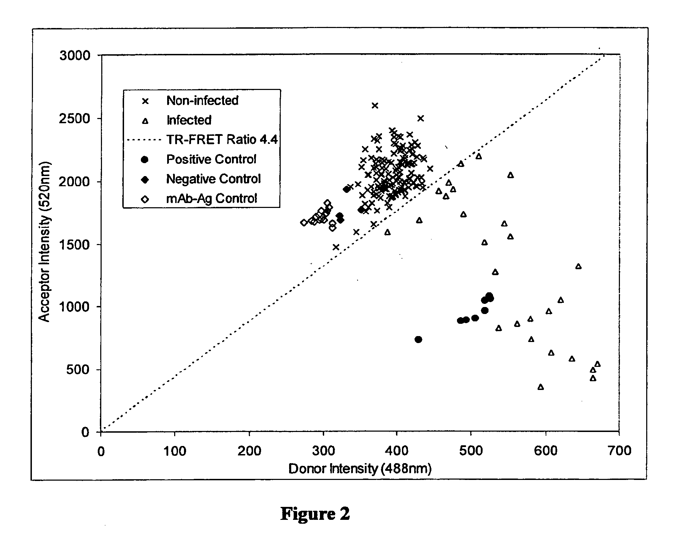

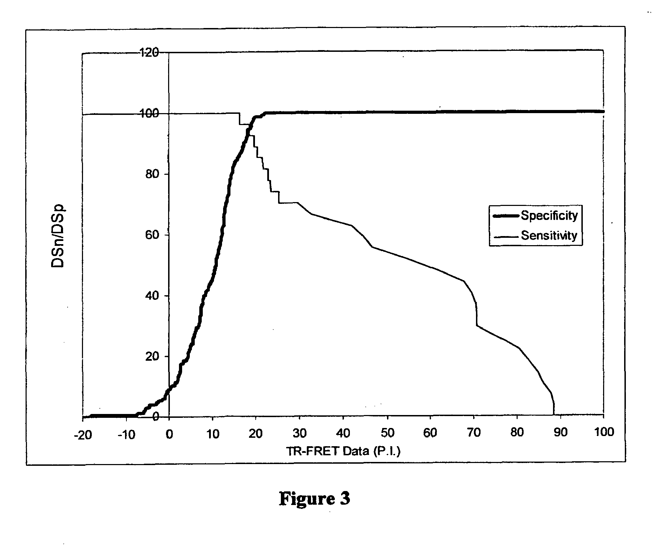

The developed assay was then validated on a small panel of serum from 153 *Brucella* non-infected and 27 *Brucella* infected cattle. The TR-FRET assay was read after 10 minutes test incubation. A test positive/negative cut-off was selected which optimised the Diagnostic Specificity (DSp) and Sensitivity (DSn) of the assay. The results, shown in Figures 2 and 3, demonstrate that TR-FRET can be used for serological diagnosis of infectious disease, and with a sensitivity and specificity which is similar to that of ELISA.

The test is very simple to perform with only four reagents (antigen, antibody, serum and buffer) all added at the same step, a short test incubation period (less than 10 minutes), no separation steps and a single measurement step. The test can be performed in a 96 well microtitre plate format and the methodology is suitable for transfer to 384 or 1536 plates making the TR-FRET assay of the invention ideal for high throughput screening. It may also however be used for point of care or field use of appropriate samples, in particular where it is applied to a single sample, and the results read using a single tube fluorescence reader.

## Brief Description of Figures

The invention will now be particularly described. by way of example only, with reference to the accompanying diagrams in which:

Figure 1 is a graph showing the effect of the addition of unlabelled first binding reagent (unlabelled BM40 mAb) on an assay of the invention for the detection of antibodies to *Brucella;*

Figure 2 is a scatter graph of donor intensities against acceptor intensities for control and validation samples tested using the method of the invention;

Figure 3 is a graph showing the changes in the Diagnostic sensitivity (DSn) and specificity (DSp) of the *Brucella* TR-FRET assay as the test positive/negative cut-off is placed at different points along the range of possible PI (Percentage Inhibition) values (on the x-axis);

Figure 4 is a scatter plot of the fluorescence intensity (FI) of the donor (Terbium - 488 nm) on the x-axis against the fluorescence intensity (FI) of the acceptor (Fluorescein - 520 nm) on the y-axis after 30 mins incubation of the optimised *Brucella* TR-FRET assay;

Figure 5 is a scatter plot of the *Brucella* TR-FRET results read at 30 minutes against the results for the same samples read at 15 minutes and at 60 minutes, with the positive negative cut-off for the TR-FRET assay (120%) being shown as a dashed line;

Figure 6 is a scatter plot of the *Brucella* cELISA results (expressed as a percentage of the conjugate control) against the *Brucella* TR-FRET results read at 30 minutes, with the dashed lines representing the positive/negative cut-off for each assay;

Figure 7 is a line graph showing increasing inhibition of the *Brucella* TR-FRET signal as the concentration of *B. abortus* OIE ELISA Strong Positive Standard Serum (diluted in negative serum and whole blood preparations) increases;

Figure 8 is a line graph showing increased inhibition of the *Brucella* TR-FRET signal as the concentration of *B. melitensis* 16M cells in the test matrix increases;

Figure 9 is a line graph showing changes in the sandwich format *Brucella* TR-FRET signal in relation to the concentration of *B. melitensis* 16M cells in TBS and the concentration of labelled BM40;

Figure 10 is a line graph showing changes in the sandwich format *Brucella* TR-FRET signal in relation to the concentration of *B. melitensis* 16M cells in TBS;

Figure 11 is a line graph showing changes in the sandwich format *Brucella* TR-FRET signal in relation to the concentration of *B. melitensis* 16M cells in the test matrix;

Figure 12A is a line graph showing changes in the BVD TR-FRET signal due to the duration of incubation with unlabelled E2 antigen and the duration (5-60 mins) and method (with E2-bt and Step-Tb or WB214-FITC mAb) of pre-incubation;

Figure 12B is a line graph showing changes in the BVD TR-FRET signal due to the duration of incubation with unlabelled WB214 mAb and the duration (5-60 mins) and method (with E2-bt and Strep-Tb or WB214-FITC) of pre-incubation;

Figure 13 is a line graph showing increased inhibition of the BVD TR-FRET signal as the concentration of unlabelled E2 antigen increases and after incubation of between 5-90 mins (excluding a 5 mins pre-incubation of the unlabelled E2 with WB214-FITC);

Figure 14 is a line graph showing increased inhibition of the BVD TR-FRET signal as the concentration of unlabelled WB214 mAb increases and after incubation of between 5-90 mins (excluding a 5 mins pre-incubation of the unlabelled WB214 with E2-bt and Strep-Tb antigen);

Figure 15 is a scatter plot of BVD TR-FRET results, after 5 mins incubation with labelled E2 and WB214 mAb (and a 5 mins pre-incubation with E2-bt and Strep-Tb) against anti-BVD antibody iELISA results in which high iELISA results are representative of high antibody titre as are low TR-FRET results; and

Figure 16 is a scatter plot of BVD TR-FRET results, after 60 mins incubation with labelled E2 and WB214 mAb (and a 5 mins pre-incubation with E2-bt and Strep-Tb) against anti-BVD antibody iELISA results in which high iELISA results are representative of high antibody titre as are low TR-FRET results.

## Examples

### Example 1- Diagnosis of Brucellosis

The applicants developed a TR-FRET protocol as described below. The method was used to analyse samples from *Brucella* infected and uninfected cattle and the results are illustrated below.

#### Antibody labelling with Terbium

The BM40 mAb used was a mouse $IgG_1$ antibody specific to *Brucella* 'M' O-antigen epitopes (Greiser-Wilke & Moenning, Ann Inst. Pasteur Microbiol. 1987 138 (5) 549-60). The supernatant from a BM40 producing B-cell hybridoma cell culture was affinity purified using a protein G column.

To label the antibody, 3 ml of BM40 was dialysed against sodium carbonate buffer (pH 9.5) for 21 hours at 4°C using a 1-3 ml 10 kDa Molecular Weight Cut-Off (MWCO) Slide-a-lyzer (Pierce™) dialysis cassette. The BM40 mAb was recovered from the cassettes and centrifuged in 3 kDa MWCO Centricons (Millipore, Billerica, MA) at 4000g for 90 minutes at +4°C which decreased the volume to 0.7 ml. This was spectrometrically determined to be at a concentration of 2.48 mg/ml, therefore the total amount of mAb was 1.74 mg. The terbium (Tb) chelate (100 $\mu$g) was reconstituted with 20 $\mu$l of sodium carbonate buffer (pH 9.5) and left to stand at room temperature for 5 minutes prior to the addition of the 1.74 mg of BM40 in 0.7 ml sodium carbonate buffer. After addition of the BM40 mAb, the container was wrapped in aluminium foil and incubated for 240 minutes at room temperature then immediately added to a 0.5-3 ml 7 kDa MWCO dialysis cassette and dialysed with 2.5 litres of de-ionised water for 48 hours. To remove any residual unbound Tb, the mAb preparation was de-salted using a Zebra™ column, MWCO 7kDa, according to the manufacturer's instructions (Pierce).

Quantification of BM40 labelling with Tb was performed spectrophotometrically. The absorbance of the terbium labelled BM40 conjugate (BM40-Tb) was measured at 280 nm and 343 nm and the concentrations of Tb-chelate and BM40 were calculated as below:

$$\text{[Tb-chelate] (M)} = (A_{343}/12{,}570) \times \text{dilution factor}$$

$$\text{[BM40] (M)} = ((A_{280}-(1.1 \times A_{343}))/210{,}000) \times \text{dilution factor}$$

When the Tb-chelate is conjugated to an amine, its extinction coefficient at 280 nm is 1.1 times its value at 343 nm. This was the basis for the derivation of the above formulae.

Antigen labelling with FITC

The antigen used was *Brucella* lipopolysaccharide (LPS) derived from *Brucella melitensis* biovar 1 strain 16M. This is a classic reference strain and is routinely used as a diagnostic antigen, in a current cELISA for example. The cells were propagated and then grown on Serum Dextrose Agar medium, incubated for 3 days at 37°C and 10% $CO_2$ for 3 days and subsequently harvested as sufficient growth had been obtained. The LPS was then extracted by the hot phenol method as described in Chapter 2.3.1 of the OIE Manual of Diagnostic Tests and Vaccines for Terrestrial Animals (5th edition, 2004) which is based upon the method of Whestphal et al., 1952. At the end of the production process the LPS antigen was freeze dried in small aliquots of approximately 3 mg and stored at +4°C until use.

The *Brucella* 16M smooth LPS antigen was labelled using Fluorescein Isothianocynate (FITC) Isomer 1 (Sigma). Each vial of 16M LPS antigen (3 mg) was dissolved in 600 $\mu$l of 0.1 N NaOH (BDH Prod.) and incubated at 37°C for 1 hour. Then 300 $\mu$l of a freshly prepared solution of FITC in DMSO (Sigma at 50 mg/ml was added, mixed well, and incubated for 2 hours at 37°C. After this period, the antigen was immediately desalted twice using Zebra™ columns, 7 kDa MWCO, according to the manufacturers' instructions (Pierce).

Test Method

A panel of 153 sera from 153 non-infected bovines were tested by TR-FRET. A panel of 27 sera from 27 bovines with brucellosis were also tested. These samples were either confirmed as infected by cultural identification of *Brucella* or were serologically positive (by classical serology e.g. CFT and SAT) and from herds from which *Brucella* had been cultured.

The BM40-Tb labelled antibody was used at a final concentration of 16nM. The *B. melitensis* 16M sLPS FITC labelled antigen (*Brucella* sLPS-FITC) was used at a final concentration of 1/64, and the serum was tested at a final concentration of 1/21. Dilutions were made in TBS test buffer (Tris-buffered Saline pH 7.4 (0.05 M Tris (Sigma) & 0.15 M NaCl (BDH) adjusted to pH 7.4 with HCl (BDH).

A stock volume of 2 x concentrate of the BM40-Tb was prepared and 100 $\mu$l of this was added to each test well of a black 96 well microtitre plate (Costar, flat bottom, non-treated, non-sterile, black polystyrene, from Corning Incorporated, NY 14831). Then 5 $\mu$l of test/control serum was added to each of the wells in duplicate. Then 100 $\mu$l of a 2 x concentrate of the 16M FITC antigen was then added to all wells. The plates were incubated on the bench at room temperature for 10 minutes and then read by a Tecan GENios Pro as described below.

Four control types were used on each of the test plates. Three serum controls were used, these were two control samples from the *Brucella* cELISA: the Strong positive (Goat Serum 8/55/7), and the Negative (Sheep Serum SSN02/07), and the Positive control used in the *Brucella* bovine iELISA. The fourth control contained only buffer, BM40-Tb and 16M FITC. This Uninhibited control represented the maximal, uninhibited TR-FRET level of the reagents used.

The reagents in each test well were read by the plate reader which measures the intensity of the light emitted at 488 nm (10 nm bandwidth) and 520 nm (10 nm bandwidth) following excitation with light at 340 nm (60 nm bandwidth). A time delay of 100 $\mu$s after the excitation was set before the initiation of the emission measurements. Following this delay, the emission was then measured for a period of 200 $\mu$s. The raw fluorescence intensity data was then converted to a ratio value by dividing the 520 nm value by the 488 nm value for each test well. The 520 nm emission intensity values are due to acceptor emission whereas the 488 nm values are from the donor. Therefore large ratio values indicate that energy transfer has occurred whereas low ratio values indicate that it has not. The Uninhibited control samples represent the theoretical maximum energy transfer (i.e. 100%) that can take place between the donor and acceptor in this system. All the ratio values are normalised by calculating each as a percentage of this system maximum value. The difference between the Uninhibited control (100%) and the test sample percentages is the percentage inhibition (PI) as this demonstrates the degree to which the test sample has inhibited TR-FRET.

Plate Reader Settings

For Terbium excitation a 340 nm filter with a 60 nm bandwidth was selected (Tecan part No. 30000349). For measurement of Terbium emission a 488 nm filter with a 10 nm bandwidth was selected (Tecan part No. 30000451). For measurement of FITC emission a 520 nm filter with a 10 nm bandwidth was selected (Tecan part No. 30000463). These were installed into a Tecan GENios Pro according to the manufacturers' instructions. The plates were read with the Lag and Integration times set to 100 and 200 $\mu$s respectively.

Results

The graph shown in Figure 1 illustrates the effect of the addition of unlabelled BM40 monoclonal antibody to working strength concentrations of BM40-Tb and FITC labelled 16M LPS. The unlabelled BM40 competed with the BM40-Tb for binding sites on the labelled 16M LPS. This competition results in a decrease in acceptor fluorescence, as the donor and acceptor fluorophores become separated, and an increase in the donor fluorescence. This in turn causes the reduction in the TR-FRET ratio (520 nm intensity / 488 nm intensity) from approximately 6 to 0.5. As can be seen from the graph, these affects are dose dependent. These results demonstrate that the *Brucella* TR-FRET assay can detect the addition of competing antibodies through changes in the TR-FRET ratio.

Illustrative results for positive and negative samples are shown in Figure 2. The graph shows the raw data from the 153 samples from non-*Brucella* infected bovines and 27 samples from *Brucella* infected bovines. It also shows the data from the test controls: four Positive goat control replicates, four Positive bovine control replicates, four Negative control replicates and 16 Uninhibited control replicates containing BM40-Tb, FITC labelled 16m LPS and test buffer only. This raw data is used to calculate the TR-FRET ratio which is the 520 nm intensity divided by the 488 nm intensity (520 nm/488 nm). The dashed line represents a TR-FRET ratio of 4.4. All samples from *Brucella* infected animals have a TR-FRET ratio less than 4.4 whereas all samples from *Brucella* non-infected animals have a TR-FRET ratio greater than 4.4. The intensity values for the control samples show good reproducibly, especially for the uninhibited controls. In this format, where the test parameter is the TR-FRET ratio of the sample, the assay has 100% discrimination between the infected and non-infected samples.

The test data, expressed as percentage inhibition has been presented in Figure 3 as a Two Way Receiver Operator Curve (TW-ROC) curve. The PI results may provide a more robust and accurate test parameter than the simple ratio but either could be used.

Figure 3 shows the changes in the Diagnostic sensitivity (DSn) and specificity (DSp) of the TR-FRET assay as the test positive/negative cut-off is placed at different points along the range of possible PI values (on the x-axis). As expected, there is only a narrow range of PI values where a cut-off would generate high values for both specificity and sensitivity - this is in the region of 15-25 PI. The optimal TR-FRET test cut-off for the bovine samples is 19.7 PI This gives a DSp of 98.04% and a DSn of 92.59%.

These samples have also been tested by iELISA, cELISA and FPA. The results for these tests are shown in Table 1 below.

Table 1

|  | TR-FRET (Ratio) | TR-FRET (PI) | iELISA | cELISA | FPA |
|---|---|---|---|---|---|
| Specificity | 100.0 | 98.0 | 100.0 | 100.0 | 98.0 |
| Sensitivity | 100.0 | 92.6 | 96.3 | 96.3 | 92.6 |

In summary, this assay is flexible, rapid, homogeneous, requires no serum pre-dilution, needs only one reading and requires only one addition stage, as serum, antigen and mAb can all be added at the same time. The time required for each reading may vary for different TR-FRET assay formats, but generally, these are quick, for example from about 2 minutes. The method presents considerable labour savings compared to all other serological assays from classical techniques such as CFT, to more robust methods such as ELISA and even contemporary homogeneous assays such as the FPA and AlphaLISA. It presents advantages for both low and high throughput testing where it is probable that it will be an effective test when used as a single tube assay or when scaled down to 384 or even 1536 formats.

## Example 2 - Further studies relating to diagnosis of Brucellosis

The methods described above in Example 1 were further optimised and validated as described below. The results of further studies using the optimised protocols are also described.

### Test Method

The labelling of terbium to BM40 was improved by increasing the conjugation time to 6 hrs and removing excess unconjugated terbium by desalting with a 5ml Zebra™ column (Pierce), as described above, without prior dialysis. This improved the terbium to BM40 molar ratio to more optimal levels. The production yield of *Brucella* sLPS-FITC was improved by desalting using a PD-10 column (GE Healthcare) following the manufacturers instructions, rather than a Zebra™ column (Pierce). Titration of these reagents against control serum (see above) demonstrated optimal reagent concentrations were 2 nM BM40-Tb and a 1/1750 dilution of *Brucella* sLPS-FITC. The optimal serum sample concentration was determined to be 1/5.

The TR-FRET assay plates described above were replaced by ½ area black polystyrene non-binding surface 96 well plates (Corning No. 3686) as these improved the intensities of the fluorescent signals without increasing background readings. The lag and integration settings were optimised and as a result changed to 80 μsec (lag) and 50 μsec

(integration) from those described above.

The method of determining the *Brucella* TR-FRET assay positive/negative cut-off was adapted from that described above. A low titre positive control sample, equal to the titre of a 1/8 dilution (in negative serum) of the *B.abortus* OIE ELISA Strong Positive Standard Sera (OIEELISA$_{SP}$SS) was prepared and used in each *Brucella* TR-FRET test plate. The data for each test serum sample (520 nm fluorescence intensity / 488 nm fluorescence intensity) was expressed as a percentage of the equivalent data (520 nm fluorescence intensity / 488 nm fluorescence intensity) for the low titre positive control.

The TR-FRET assay was also demonstrated to be equally effective using either TBS or PBS as assay substrates and unaffected by low concentrations of sodium azide (as typically used to assist in reagent preservation).

To assess the diagnostic sensitivity (DSn) of the optimised *Brucella* TR-FRET assay, single serum samples (from the applicants' serum archive) from 32 cattle and 41 sheep and goats (small ruminants) were tested. Of the cattle samples, eight were from naturally infected culture positive animals, two were from culture positive animals experimentally infected with *B. abortus* strain 544, 10 were from culture positive animals, and a further 12 were from serologically positive (by CFT and SAT) animals from a culturally confirmed outbreak of brucellosis. Of the 41 small ruminant samples: two were from naturally infected culture positive animals, five were from culture positive animals from experimental infection with *B. melitensis,* nine animals were serologically positive (by CFT and SAT) and from a culturally confirmed outbreak of brucellosis and the remaining 25 animals were from a suspected outbreak of brucellosis from an endemic area.

To assess the diagnostic specificity (DSp) of the *Brucella* TR-FRET assay, single serum samples from 240 randomly selected cattle from Great Britain (officially brucellosis free since 1985) were collected. In addition, single serum samples from 240 randomly selected sheep and goats from Great Britain were also collected.

A stock volume of 5 nM concentrate of the BM40-Tb was prepared and 40 μl of this was added to each test well of a ½ area black polystyrene non-binding surface 96 well plate (Coming No. 3686). Then 20 μl of test/control serum was added to each of the wells in duplicate. Then 40 μl of a 1/700 dilution of the *Brucella* sLPS-FITC antigen was added to all wells. The plates were incubated on the bench at room temperature for 60 minutes and read at 15, 30 and 60 minutes by a Tecan GENios Pro using the optimised settings as described above.

Four control types were used on each of the test plates. Three serum controls were used, these were two control samples from the VLA *Brucella* cELISA (COMPELISA): the Strong Positive (Goat Serum 8/55/7), and the Negative (Sheep Serum SSN02/07), and a low titre positive control calibrated to be equal in titre (in the *Brucella* TR-FRET assay) to a 1/8 pre-dilution in negative serum of the *B. abortus* OIEELISA$_{SP}$SS. The fourth control contained only buffer, BM40-Tb and 16M FITC.

Test Results

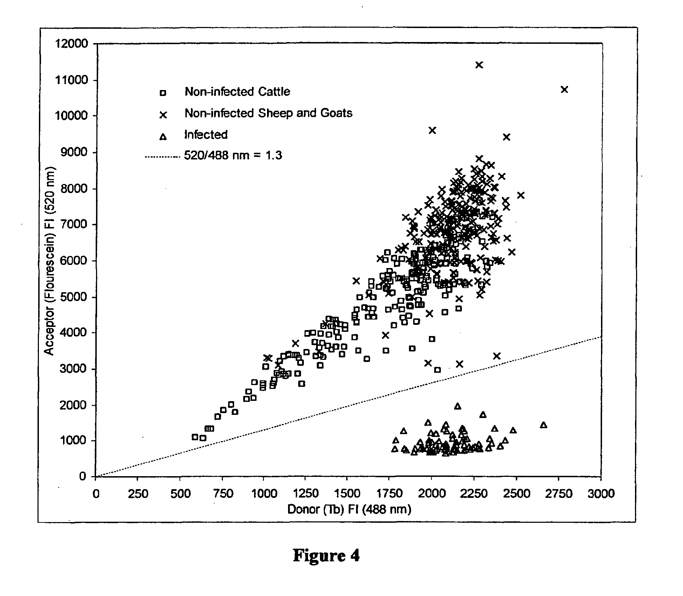

Illustrative results for samples from infected and uninfected animals are shown in Figure 4. The graph shows the raw data from the 480 samples from non-*Brucella* infected bovines and 73 samples from *Brucella* infected bovines. This raw data is used to calculate the TR-FRET ratio which is the 520 nm intensity divided by the 488 nm intensity (520 nm/488 nm). The dashed line represents a TR-FRET ratio of 1.3. All samples from *Brucella* infected animals have a TR-FRET ratio less than 1.3 whereas all samples from *Brucella* non-infected animals have a TR-FRET ratio greater than 1.3.

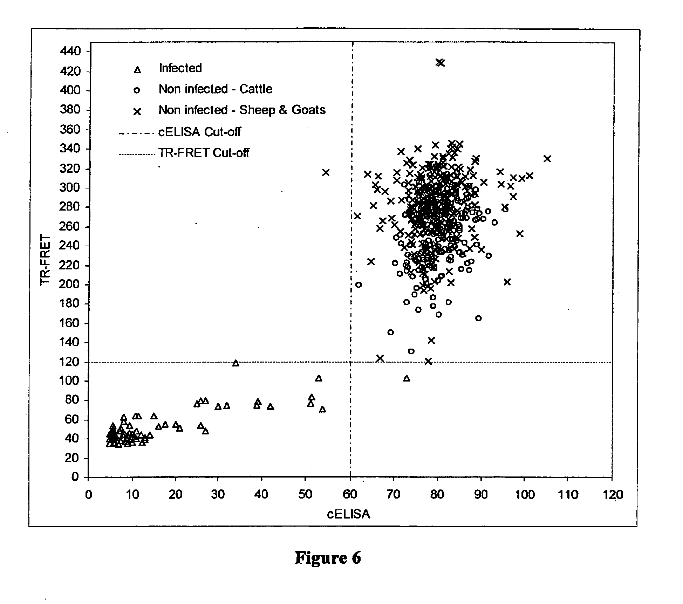

These samples have also been tested by iELISA, cELISA and FPA. The results for these tests and for cELISA are shown in the table below. The TR-FRET and cELISA results are also shown graphically in Figure 6. The *Brucella* TR-FRET results have been presented for readings taken after 15, 30 and 60 minute incubation periods. The TR-FRET had 100% DSn and DSp for cattle samples for all three incubation periods. For sheep and goat samples the *Brucella* TR-FRET had 100% DSn and DSp for the 30 and 60 minute times, the 15 minute incubation did not match these results as there was one false positive. Table 2 also shows the range of *Brucella* TR-FRET test values that can be selected as the positive/negative cut-off whilst maintaining the DSn and DSp values shown. This range grows as the incubation time increases. At 30 and 60 minutes it is possible to set the positive/negative cut-off at a test value of 120% which results in 100% DSn and DSp for cattle and sheep and goats. This is the positive/negative cut-off that has been used for the rest of the analysis.

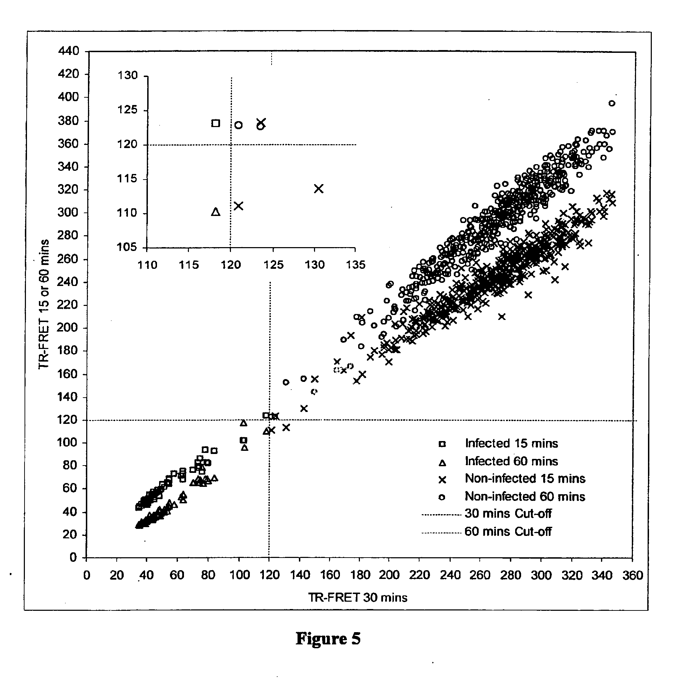

The *Brucella* TR-FRET results for all the sera in the evaluation panel are presented in Figure 5 which plots the results after 30 minutes incubation against the results for the same sample at 15 and 60 minutes. This shows there was a consistent response against time as the results all fit close to a straight line (correlation coefficients for 30 against 15, and 30 against 60 minute incubations respectively were 0.991 and 0.993). The graph also shows there was good separation between the results from the infected and the non-infected animals as they mainly fit into the bottom left and top right quadrants respectively, the quadrants having been formed by plotting the 120% cut-off value. Separation was perfect for the 30 and 60 minute data but not for the 15 minute data where there are two false positives and one false negative result with a 120% cut-off. This can be seen more closely in the magnified inset. The graph shows that generally, samples from infected animals are more positive (have higher titres) and non-infected animals more negative (have lower titres) after 60 minutes incubation compared to 15 minutes.

**Table 2.** The DSn and DSp for the *Brucella* TR-FRET assay at 15, 30, and 60 minutes is shown together with the same values obtained from the same samples using the cELISA, iELISA, and FPA. The optimal cut-off shows the range of values (of the test result) from which the positive/negative cut-off could be selected and which would provide the optimal DSn and DSp values shown. The FPA results on the cattle sera are shown where the borderline samples have been defined as negative (bl-ve) and positive (bl +ve). There is no borderline category for the sheep and goat FPA. NA means 'not applicable'.

| | *Brucella* TR-FRET | | | cELISA | iELISA | FPA | |
| | 15 mins | 30 mins | 60 mins | | | bl-ve | bl+ve |
|---|---|---|---|---|---|---|---|
| Cattle<br>DSn (n = 32) | 100.0 | 100.0 | 100.0 | 96.86 | 100.0 | 100.00 | 100.00 |
| 95% Confidence Interval | 89.06 - 100.00 | 89.06 - 100.00 | 89.06 - 100.00 | 87.34 - 99.92 | 89.06 - 100.00 | 89.06 - 100.00 | 89.06 - 100.00 |
| DSp (n = 240) | 100.0 | 100.0 | 100.0 | 100.0 | 100.0 | 96.25 | 85.00 |
| 95% Confidence Interval | 98.47 - 100.00 | 98.47 - 100.00 | 98.47 - 100.00 | 98.47 - 100.00 | 98.47 - 100.00 | 92.96 - 98.26 | 79.52 - 89.13 |
| Optimal cut-off (min-max) | 102.1 - 113.6 | 103.4 - 130.5 | 96.4 - 143.6 | NA | NA | NA | NA |
| Small Ruminants<br>DSn (n = 41) | 100.0 | 100.0 | 100.0 | 100.0 | 100.0 | 90.24 | |
| 95% Confidence Interval | 91.40-100.00 | 91.40-100.00 | 91.40 -100.00 | 91.40-100.00 | 91.40-100.00 | 76.76-97.28 | |
| DSp (n = 240) | 99.58 | 100.0 | 100.0 | 99.58 | 100.0 | 100.0100.0 | |
| 95% Confidence Interval | 97.67 - 99.99 | 98.47-100.00 | 98.47 -100.00 | 97.67 - 99.99 | 98.47-100.00 | 98.47 -100.00 | |
| Optimal cut-off (min-max) | 123.1 - 123.2 | 118.2 - 120.9 | 117.5 - 122.6 | NA | NA | NA | |

Detection of antibodies to *Brucella* sLPS in whole blood preparations

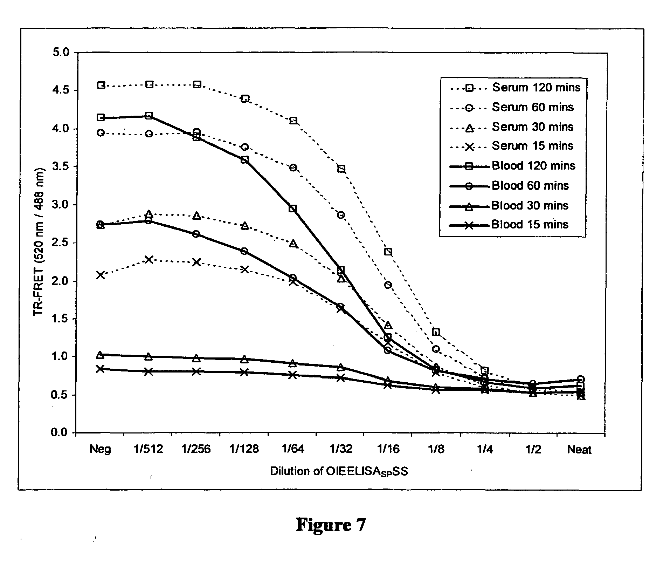

The competitive *Brucella* TR-FRET method described above was also applied to the detection of antibodies against *Brucella* sLPS in samples prepared using whole blood. A whole blood sample from an uninfected cow was separated into the plasma and cellular components by centrifugation. The plasma was removed and replaced with an equal amount of negative bovine serum containing varied dilutions of the OIEELISA$_{SP}$SS (in double dilutions ranging from neat to 1/512) as described previously. The samples were then mixed to ensure homogeneity of the preparation. The TR-FRET test was then performed on this sample by adding 40 $\mu$l of the whole blood preparation containing the dilution of the OIEELISA$_{SP}$SS to the test plate (the same as previously described), 30 $\mu$l of BM40-Tb and 30 $\mu$l of *Brucella* sLPS-FITC such that the latter components were at the same final working strength concentrations as described for the serum assay. By way of a comparison, the OIEELISA$_{SP}$SS dilutions used to inoculate the blood cells were also tested by TR-FRET according to the serological testing protocol described previously. The plate was then left on the bench at room temperature and the TR-FRET results for all samples were read (as described previously) at 15, 30, 60 and 120 minutes. The results are shown in Figure 7.

The results show that the *Brucella* TR-FRET assays does detect the presence of anti-*Brucella* sLPS antibodies in preparations of whole blood. This detection does take longer in such preparations compared with the detection of the same antibodies in serum. The antibody detection in whole blood preparations is approximately one double dilution less sensitive than in serum. Nevertheless, the results do show that the *Brucella* TR-FRET assay works directly with whole blood preparations. The difference between this preparation and whole blood is that whole blood will also contain clotting factors such as fibrinogen.

Detection of *Brucella* cells in PBS, serum and milk using the competitive *Brucella* TR-FRET method

The competitive *Brucella* TR-FRET described above can be used to detect antibodies and antigens that react to either labelled component in the assay. To demonstrate this, and the ability of the *Brucella* TR-FRET to work directly with whole milk samples, PBS and whole milk was inoculated with a dilution series of heat killed *Brucella* 16M whole cells as measured in colony forming units (CFUs).

*B. melitensis* strain 16M cells were grown on serum dextrose agar plates for 5 days at 10% $CO_2$ and 37°C and then harvested into sterile PBS. The cell content was quantified by counting *Brucella* colonies on serum dextrose agar plates inoculated with a known volume from a dilution series to of the antigen and incubated for 5 days at 10% $CO_2$ and 37°c. These results enabled the determination of the concentration of *B. melitensis* strain 16M cells to be expressed in colony forming units (CFUs) per ml. The cells were heat killed by incubation at 80°c for 10 hrs prior to use.

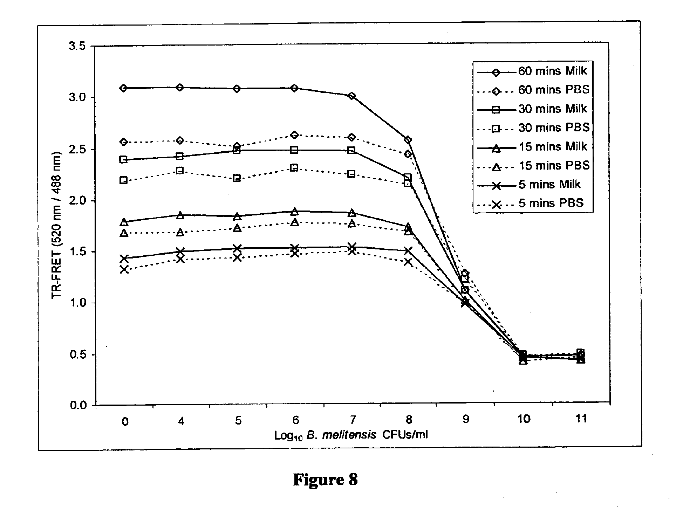

The *Brucella* TR-FRET assay was performed by adding 50 µl of the inoculated PBS or whole milk sample was added to 25 µl of BM40-Tb and 25 µl of *Brucella* sLPS-FITC labelled 16M sLPS (diluted in PBS). The final concentrations of the labelled reagents were as described for the competitive TR-FRET assay described previously and the final concentrations of *B. melitensis* 16M whole cells are as shown in figure 8.

The data shown in Figure 8 demonstrates that the competitive *Brucella* TR-FRET assay can detect the presence of *Brucella* 16M whole cells even after only 5 minutes incubation with the complete reagent set. The detection limit of the assay is between $10^8$ and $10^7$ *Brucella* CFUs/ml. It also shows that this is possible within 50 µl of PBS and 50 µl of whole milk. The difference between the TR-FRET ratios of high and low *Brucella* 16M concentrations increases with incubation time. Even so, the shape of the dose response curve is similar for all incubation periods such that after 5 minutes the differences in TR-FRET ratio are evident and reproducible.

Detection of *Brucella* cells in milk, serum and culture media using sandwich TR-FRET method

The competitive *Brucella* TR-FRET protocol described above was adapted to demonstrate the capability of the method to detect *Brucella* antigens by a sandwich assay forma. In this embodiment of the technique two populations of the BM40 monoclonal antibody (as described above) were prepared. The use of two differently labelled populations of the same monoclonal antibody is possible in this circumstance owing to the presence of multiple epitopes on the analyte - in this case the *Brucella* cell.

The first population was labelled with terbium donor fluorophore as described above. The second population was labelled with FITC. This labelling was performed by adding 8 µl of FITC in DMSO (at 5 µg/µl) to 1 ml BM40 in sodium carbonate buffer pH 9.5 (at 1 mg/ml). This was incubated in the dark at 21°C for 4 hours on a rotary shaker. After this period the unbound FITC was separated from the BM40 conjugated FITC using a Zebra desalting column (Pierce) in accordance with the manufacturers' instructions. The 1 ml of reagent mixture was desalted and buffer exchanged into 50 mM Tris.HCl, 150 mM NaCl pH 7.4 by centrifugation with a 5 ml Zebra™ desalting column (Pierce) in accordance with the manufacturers' instructions. The concentration and molar ratio of the FITC labelled BM40 monoclonal antibody (BM40-FITC) was examined by spectrophotometer.

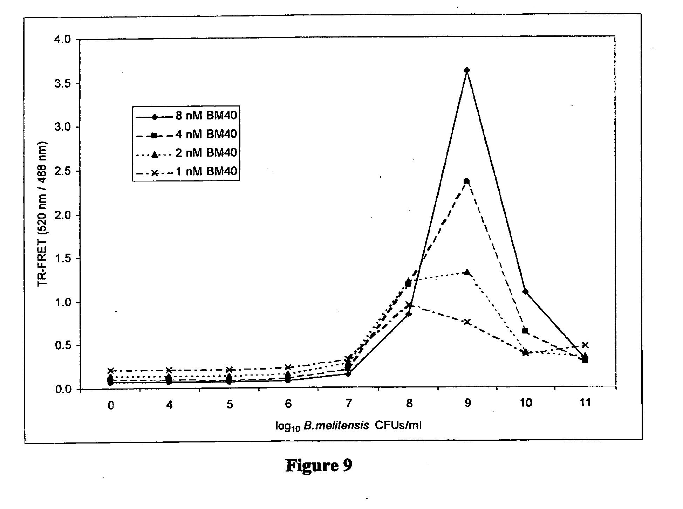

A range of concentrations of BM40-Tb and BM40-FITC (the relative concentration of the BM40-Tb mAb and the BM40-FITC mAb was always equal) were added to a half area 96 well microtitre plate (as described above) with a dilution range of heat killed *B. melitensis* 16M cells within 50 mM Tris.HCl, 150 mM NaCl pH 7.4. TR-FRET readings were taken at 5, 30 and 60 minutes using the same parameters as described for the optimised protocol described above. The results, shown in Figure 9, demonstrate that all the BM40 antibody concentrations used detected *B. melitensis* cells up to a limit of between $10^7$ and $10^6$ CFUs/ml after 30 mins incubation. The maximum TR-FRET signal occurred at $10^9$ CFUs/ml and then receded as the reaction became over saturated with antigen. Based on these results, the optimal concentration of BM40-Tb and BM40-FITC chosen for further study was 4 nM. Although the maximum TR-FRET signal with 4 nM BM40 (Tb/FITC) was not as great as for 8 nM the analytical sensitivity (as seen at $10^7$ CFUs/ml) appeared marginally superior.

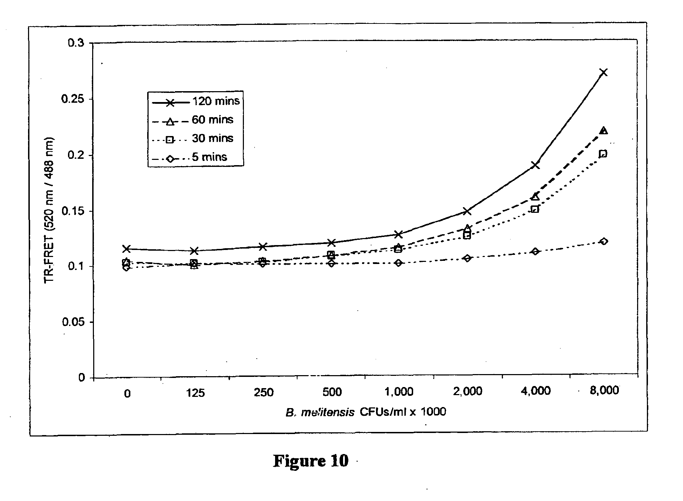

The *Brucella* sandwich (sw) TR-FRET, using 4 nM BM40, was assessed with a more focused dilution series of *B. melitensis* 16M cells in TBS and the results are shown in Figure 10. These results show that the analytical sensitivity of the method is between $2\times10^6$ and $1\times10^6$ CFUs/ml (final concentration in the test well) although an incubation period greater than 5 minutes is required. A 30 minute incubation period was effective.

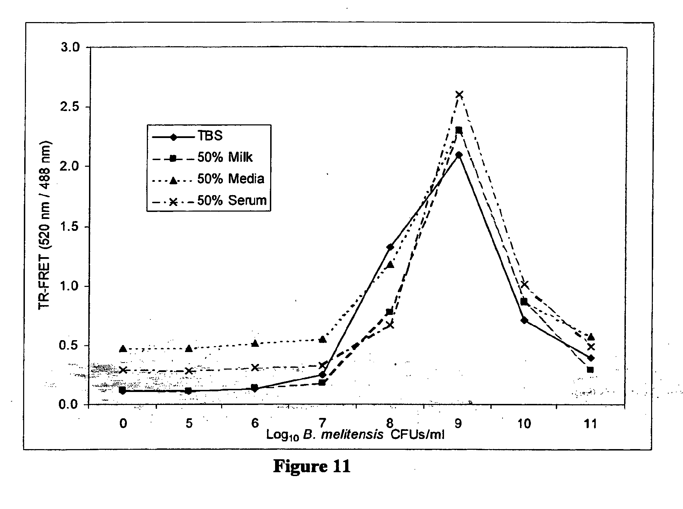

The effectiveness of this *Brucella* swTR-FRET method for detecting *B. melitensis* 16M cells in whole milk, bovine serum and *Brucella* liquid culture media (Brodie and Sintons' media) was tested by replacing 50 µl of TBS test buffer with 50 µl of these mediums in the final 100 µl test volume. The remaining 50 µl contained sufficient reagents to make up to a final concentration of 4 nM for the BM40 and the dilutions of *B. melitensis* 16M cells shown in Figure 11. These results show that the assay can detect *B. melitensis* 16M cells in all of these types of mediums with analytical sensitivities between $10^8$ and $10^6$ CFUs/ml. Once again, increasing antigen concentration to $10^{11}$ CFUs/ml leads to a decline in the TR-FRET ratio below the maximal figure suggesting that the antigen has reached concentrations such that epitopes are present in such quantities that the BM40 antibodies are more often too far apart for TR-FRET to occur.

## Example 3 - Detection of diagnostic moieties for Bovine Viral Diarrhoea (BVD) by TR-FRET

The applicants developed TR-FRET protocols as described below. The method was used to analyse samples containing anti-BVD antibodies and BVD viral antigens.

### Development of competitive TR-FRET method

The BVD TR-FRET method was developed using the following reagents in a competitive format: biotinylated recombinant BVD E2 antigen, terbium conjugated streptavidin and fluorescein conjugated anti-E2 monoclonal antibody WB214.

Production of recombinant baculovirus expressing the E2 glycoprotein for BVDV type 1a (strain C24V) was achieved by firstly cloning the region of the bovine viral diarrhoea virus genome delineated by primers BVDV C24V E2 EcoRI and BVDV C24V E2 6His XhoI (Amin Asfor PhD thesis; RVC, University of London, 2006) into the general cloning vector pGEM-T easy (Promega). The primers introduced an EcoRI site, a start codon and a Kozak consensus sequence at the 5' terminus of the construct and an XhoI site and stop codon (TAA) downstream of 6 histidine codons at the 3' terminus of the construct. Following digestion with EcoRI and XhoI the insert from the general cloning vector was subsequently cloned into the baculovirus transfer vector pBacPAK9 prior to recombination in insect cells (*Sf9* cells) with BacPAK6 baculoviral DNA. In order to purify recombinant E2 protein from the insect cell culture medium *Sf9* cells were grown to a density of 2x $10^6$ cells per ml in suspension prior to infection with recombinant baculovirus to an m.o.i. of 5-10. Flasks were incubated for a further 72 hours at a temperature of 28°C prior to harvesting the cells and spent medium in 50ml aliquots. This material was centrifuged at low speed and the supernatant used as starting material for purification under native conditions using Ni-NTA column chromatography following manufacturer's instructions (QIAGEN).

The recombinant E2 was labelled with biotin using No-Weigh™ Sulfo-NHS-Biotin (Pierce) and following the manufacturers' instructions. Unincorporated biotin was removed and the biotinylated E2 exchanged into 20 mM Tris·HCL 50 mM NaCl pH. 7.8 using a 0.5 ml Zebra™ desalting column (Pierce) in accordance with the manufacturer's instructions. The concentration of biotinylated E2 (E2-bt) was measured using BCA protein assay (Pierce).

The WB214 anti-E2 monclonal antibody was conjugated to fluorescein by adding FITC (diluted in DMSO to a concentration of 5 µg/µl) to WB214 (diluted in sodium carbonate buffer pH 9.5 to a concentration of 1 mg/ml) in a 1:10 ratio by weight. The reaction was then left to progress for 2.5 hrs at room temperature whilst shielded from light. The unincorporated FITC was removed and the conjugated antibody buffer exchanged into 50 mM Tris·HCl 150 mM NaCl pH 7.4 using a 2 ml Zebra™ desalting column (Pierce) in accordance with the manufacturers' instructions. The concentration and molar ratio of the FITC labelled WB214 monoclonal antibody (WB214-FITC) was examined by spectrophotometer.

The terbium conjugated streptavidin (Strep-Tb) was purchased from Invitrogen (product No. PV3965).

The concentrations of the three reagents for use in the BVD TR-FRET assay were optimised by checkerboard titration. The BVD TR-FRET assay was performed using the same tests plates, plate reader, filters, lag and integration times as described above for the optimised *Brucella* TR-FRET assays. The optimised reagent concentrations in the 100 µl final BVD TR-FRET assay volume were 0.5 µg/ml of E2-bt, 8 nM WB214-FITC and 10 nM Strep-Tb. In all protocols the E2-bt and Strep-Tb were mixed prior to addition to the test wells and added in a single step.

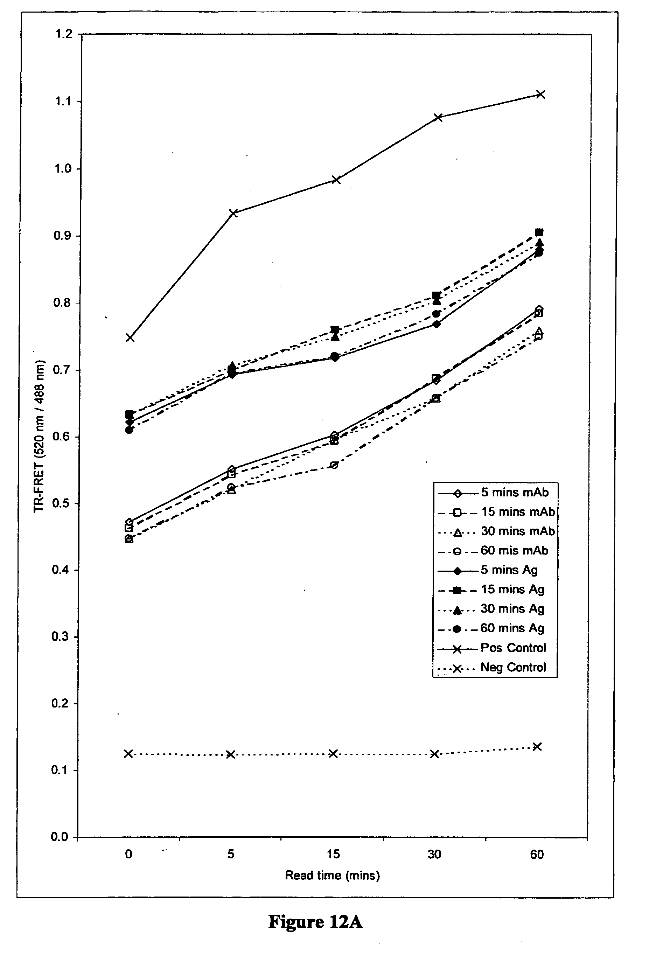

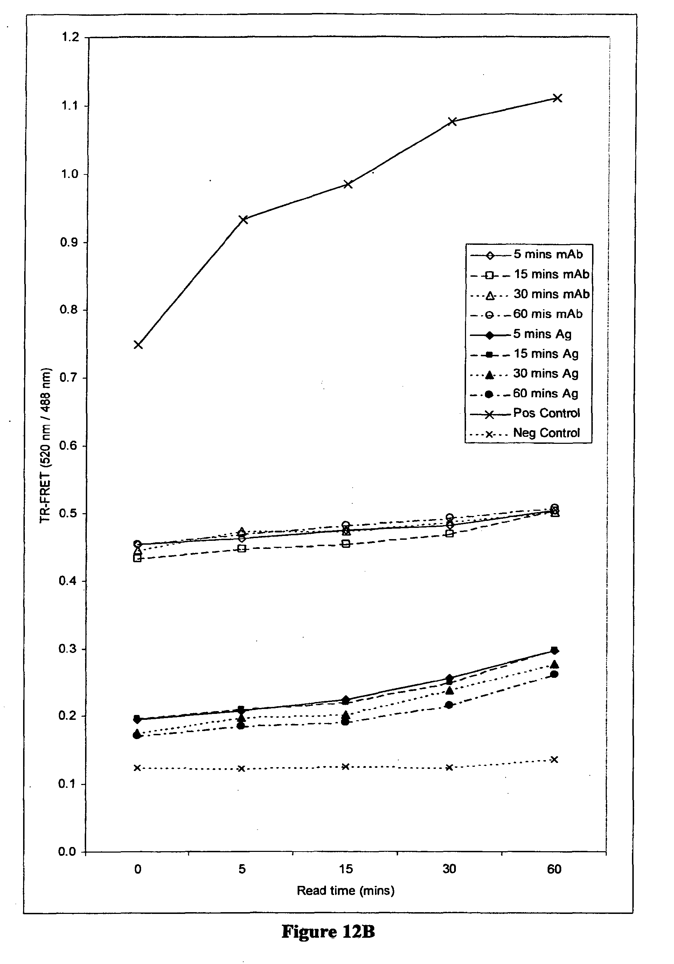

The order and timing or reagent addition to the BVD TR-FRET was also optimised for the detection of antigens or antibodies. Detection capability was assessed using unlabelled E2 antigen and unlabelled WB214 mAb. To investigate possible increases in sensitivity due to pre-incubation of the target analyte with the heterologous labelled partner (e.g. unlabelled E2 pre-incubation with WB214-FITC and unlabelled WB214 pre-incubation with E2-bt and Strep-Tb) single concentrations of target analyte were selected (0.5 µg/ml for E2 and 8 nM for WB214). Both analytes were tested with 5, 15, 30 and 60 minutes of heterologous and homologous incubation prior to the addition of the remaining reagents. The test wells were then read at varying periods of time. The results are shown in Figures 12A and 12B.

The data from Figures 12A and 12B shows that the addition of the unlabelled competing agents always reduced the BVD TR-FRET values compared to the zero inhibition control (un-inhibited TR-FRET where all the labelled reagents are used at working strength without any competing agents) but all results remain above the background (diffusion enhanced TR-FRET) control (the same as the zero inhibition control but without labelled E2 antigen). The figures also show that the BVD TR-FRET results increase with increasing read timer Figure 12A shows that pre-incubation with WB214-FITC increases the sensitivity of unlabelled E2 detection compared with pre-incubation with E2-bt and Strep-Tb. The graph also shows that longer periods of pre-incubation with WB214-FITC result in increased sensitivity for unlabelled E2. There is no such pattern regarding the duration of the incubation time with E2-bt. It is reasonable to conclude that no reaction takes place in homologous pre-incubation and that the reaction only starts once the complementary heterologous reagent is added. Therefore the difference between the homologous pre-incubation and the 5 minute heterologous pre-incubation is due entirely to this 5 minute incubation period. Compared to the effect of the 5 minute heterologous pre-incubation the effects of longer incubation periods are insubstantial. The same pattern of data was produced for the corresponding detection of unlabelled WB214 as shown in Figure 12B. Owing to this data and the conclusions taken from it, subsequent BVD TR-FRET assays to detect antigen employed a 5 minute pre-incubation with WB214-FITC and subsequent BVD TR-FRET assays to detect antibodies employed a 5 minute pre-incubation with E2-bt and Strep-Tb.

<u>Detection of anti-viral antigen monoclonal antibody</u>

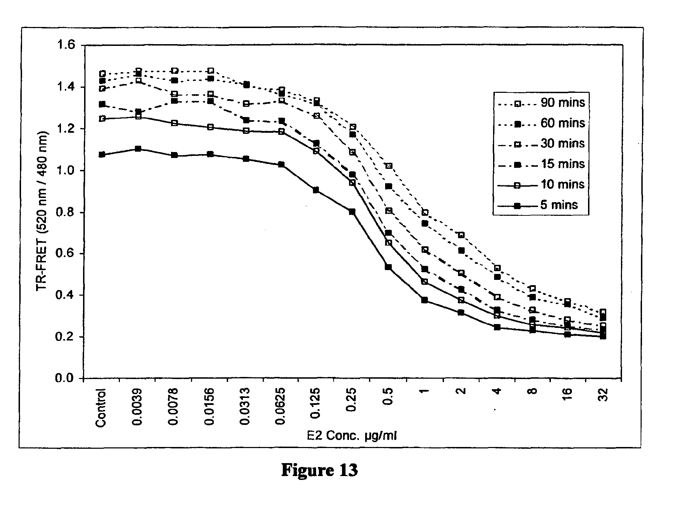

The ability to detect unlabelled WB214 was tested by adding a dilution series of the unlabelled antibody to the BVD TR-FRET assay as shown in Figure 13. These results show that the addition of increasing concentrations of the unlabelled WB214 leads to inhibition of TR-FRET. The data suggests that the assay is capable of detecting a 1 nM concentration of unlabelled WB214 and that this capability is apparent from a test incubation time of 5 minutes upwards.

<u>Detection of viral antigen</u>

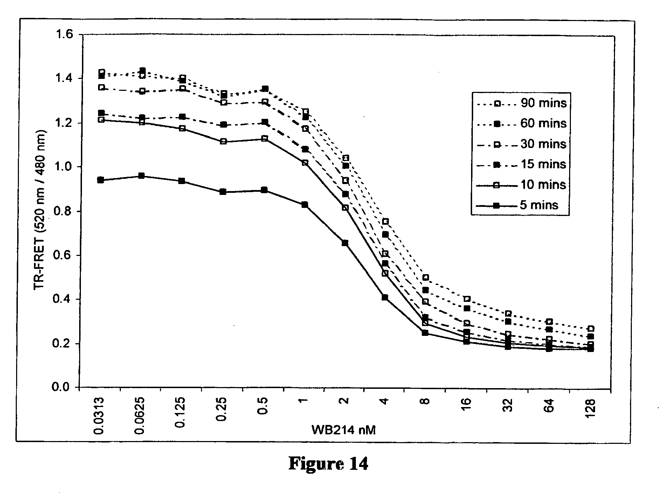

The ability to detect vial antigen was assessed by adding a dilution series of unlabelled E2 antigen to the BVD TR-FRET assay as shown in Figure 14. These results show that the addition of increasing concentrations of the unlabelled E2 leads to inhibition of TR-FRET. The data suggests that the assay is capable of detecting a 0.0625 $\mu$g/ml concentration of unlabelled WB214 and that this capability is apparent from a test incubation time of 5 minutes upwards.

<u>Detection of polyclonal anti-viral antibodies in serum samples from infected hosts</u>

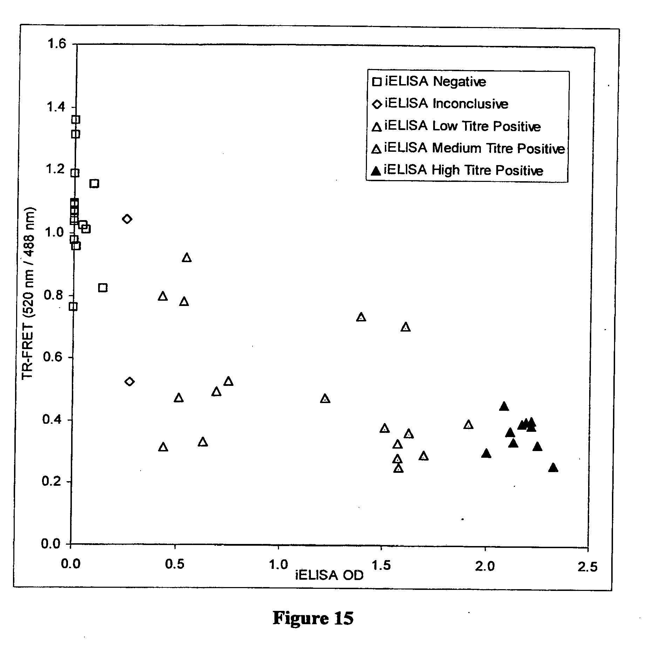

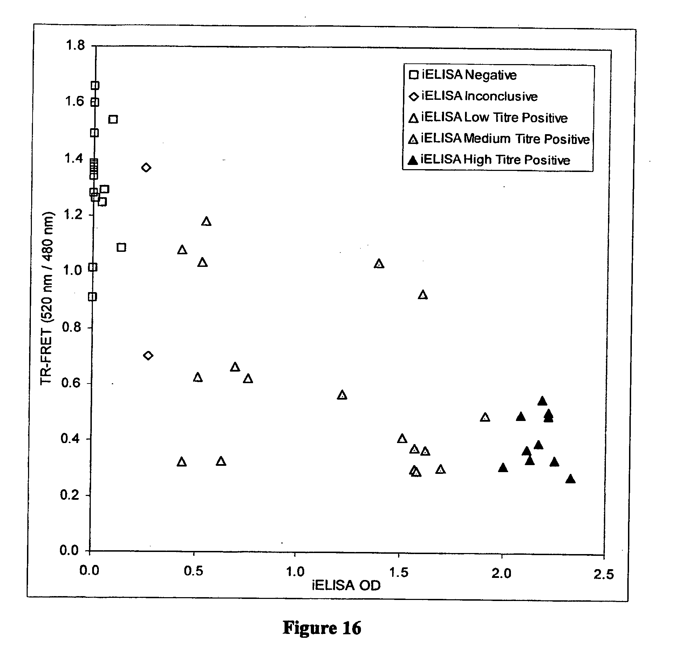

The ability of the BVD TR-FRET assay to detect anti-BVD antibodies was assessed by testing 46 bovine serum samples from 46 individual animals that had also been tested by the HerdChek BVDV Antibody Test Kit (IDEXX) following the manufacturers' instructions. BVDV Antibody Test Kit (IDEXX). The TR-FRET assay was performed by adding 40 $\mu$l of serum to the well of the test plate followed by 30 $\mu$l of E2-bt and Strep-Tb. The test plate was then left for a 5 minute pre-incubation at room temperature on the bench. After this time 30 $\mu$l of WB214-FITC was added to each well. A zero inhibition control was prepared using the test reagents and replacing the 40 $\mu$l of serum with 40 $\mu$l of test buffer (PBS). A background (diffusion enhanced) TR-FRET control was prepared the same way as the zero inhibition control but without adding the E2-bt (volume replaced with PBS). The plates were read after 5, 15, 30 and 60 minutes (excluding pre-incubation period).
The results of the BVD TR-FRET and the IDEXX iELISA are shown in Figures 15 and 16. The data shows that for samples with a high iELISA result there is a low TR-FRET result as might be expected as polyclonal serum antibodies to E2 inhibit the biding of WB214-FITC to E2-bt and therefore inhibit TR-FRET. Samples with a low iELISA result generally have a high TR-FRET result as would also be expected if there are no competing serum antibodies. There is a highly significant negative correlation between the TR-FRET result and the iELISA result after just 5 minutes incubation (r = -0.823, P < 0.001) which is consistent with the action of specific anti-BVD antibodies present in the serum inhibiting TR-FRET. It is not possible to set a cut-off for the TR-FRET that gives 100% comparative diagnostic sensitivity and specificity. The optimal comparative diagnostic sensitivity and specificity of the TR-FRET compared to the IDEXX iELISA are 87.5% and 96.4% respectively.
Although the data shows that the TR-FRET can detect BVD specific polyclonal serum antibodies it is not a homogeneous homologue of the IDEXX assay. The two tests will not detect exactly the same anti-BVD antibody populations. As such differences between the test results are not only attributable to the TR-FRET aspect of the BVD TR-FRET assay but also by its competitive nature and the antigen and monoclonal antibody used.

**Claims**

1.   A method for detecting the presence of a diagnostic moiety indicative of exposure to a *Brucella* species infectious organism in a biological sample taken from a human or animal, said method comprising;

     a) adding to said sample a first fluorescently labelled reagent which binds said diagnostic moiety, and a second fluorescently labelled reagent which either binds said diagnostic moiety in addition to said first fluorescently labelled reagent, or which binds the first fluorescently labelled reagent or a complex comprising the first fluorescently labelled reagent in competition to the said diagnostic moiety, wherein a label on one of the first or second fluorescently labelled reagents acts as a fluorescent energy donor compound and wherein the other of the first or second fluorescently labelled reagent acts as a fluorescent energy acceptor compound which is able to accept fluorescent energy from said donor compound;

b) exciting the fluorescent energy donor compound by illuminating with light of a wavelength which is absorbed by said fluorescent energy donor compound;

c) measuring fluorescent signal emitted by said fluorescent energy acceptor compound as a result of its absorption of the fluorescent energy from the donor compound after a time delay; and

d) relating the results to the presence or absence of diagnostic moiety in said sample;

wherein the diagnostic moiety is selected from the *Brucella* species infectious organism, an antigen of the *Brucella* species infectious organism, or an antibody to an antigen of the *Brucella* species infectious organism, and wherein the first and second fluorescently labelled reagents are specific binding reagents.

2. A method according to claim 1 wherein the second fluorescently labelled reagent binds the first fluorescently labelled reagent in competition to the said diagnostic moiety, and wherein a reduction in the fluorescent signal measured in step (c) is indicative of the presence of diagnostic moiety in the sample.

3. A method according to claim 1 wherein the second fluorescently labelled reagent binds a complex comprising the first fluorescently labelled reagent and a substrate in competition to the said diagnostic moiety, and wherein the absence or substantial absence of a fluorescent signal measured in step (c) is indicative of the presence of diagnostic moiety in the sample.

4. A method according to claim 1 wherein the second fluorescently labelled reagent binds said diagnostic moiety in addition to said first fluorescently labelled reagent, and wherein the increase or substantial increase of a fluorescent signal measured in step (c) is indicative of the presence of diagnostic moiety in the sample.

5. A method according to any one of the preceding claims wherein the fluorescent signal from the fluorescent energy donor compound is also measured and the ratio of the two signals is used to determine the presence or absence of diagnostic moiety in the sample.

6. A method according to any preceding claim wherein one of the first or second fluorescently labelled reagents is a *Brucella* species glycoconjugate.

7. A method according to any preceding claim wherein the one of the first or second fluorescently labelled reagents is an LPS antigen of a *Brucella* species, and the other is an antibody which binds said antigen.

8. A method according to claim 7 wherein the antigen is an O-antigen of *Brucella.*

9. A method according to any one of the preceding claims where the fluorescent energy donor compound is a lanthanide.

10. A method according to any one of the preceding claims wherein the fluorescent energy donor compound is a terbium lanthanide chelate, and the fluorescent energy acceptor compound is fluorescein or a derivative thereof.

11. A method according to any one of claims 1 to 9 wherein the fluorescent energy donor compound is a europium lanthanide chelate and the fluorescent energy acceptor compound is Cy5, allophycocyanin (APC) or an Alexa Fluor dye.

12. A method according to any one of the preceding claims wherein the biological sample is a blood, serum, plasma, milk, urine, mucous, cerebrospinal fluid, faecal or a tissue biopsy sample.

13. A method according to any one of the preceding claims which is carried out on multiple samples simultaneously in separate reaction wells.

**Patentansprüche**

1. Verfahren zum Nachweis der Anwesenheit einer diagnostischen Einheit, die bei einer aus einem Menschen oder einem Tier gewonnenen biologischen Probe auf die Exposition gegenüber einem infektiösen Organismus in Form einer Brucella-Spezies hinweist, wobei das Verfahren umfasst:

   a) Zusetzen eines ersten fluoreszenzmarkierten Reagens zu der Probe, das die diagnostische Einheit bindet, und eines zweiten fluoreszenzmarkierten Reagens, das entweder die diagnostische Einheit zusätzlich zu dem ersten fluoreszenzmarklerten Reagens bindet oder das erste fluoreszenzmarkierte Reagens oder einen das erste fluoreszenzmarkierte Reagens enthaltenden Komplex kompetitiv zur diagnostischen Einheit bindet, wobei eine Markierung an einem der ersten oder zweiten fluoreszenzmarkierten Reagentien als Fluoreszenzenergie-Donorverbindung agiert und wobei das andere der ersten oder zweiten fluoreszenzmarkierten Reagentien als Fluoreszenzenergie-Akzeptorverbindung agiert, die Fluoreszenzenergie von der Donorverbindung empfangen kann;
   b) Anregen der Fluoreszenzenenergie-Donorverbindung durch Beleuchten mit Licht mit einer Wellenlänge, die von der Fluoreszenzenergie-Donorverbindung absorbiert wird;
   c) nach einer zeitlichen Verzögerung erfolgendes Messen des Fluoreszenzsignals, das von der Fluoreszenzenergie-Akzeptorverbindung ausgestrahlt wird, nachdem diese die Fluoreszenzenergie von der Donorverbindung aufgenommen hat; und
   d) Verknüpfen der Ergebnisse mit der Anwesenheit oder Abwesenheit der diagnostischen Einheit in der Probe;

   wobei die diagnostische Einheit ausgewählt ist aus dem infektiösen Organismus in Form einer *Brucella*-Spezies, einem Antigen des infektiösen Organismus in Form einer *Brucella*-Spezies oder einem Antikörper gegen ein Antigen des infektiösen Organismus in Form einer *Brucella*-Spezies, und , wobei die ersten und zweiten fluoreszenzmarkierten Reagenzien spezifische Bindungsreagenzien sind.

2. Verfahren nach Anspruch 1, wobei das zweite fluoreszenzmarkierte Reagens das erste fluoreszenzmarkierte Reagens kompetitiv zur diagnostischen Einheit bindet und wobei ein in Schritt (c) gemessener Rückgang des Fluoreszenzsignals auf die Anwesenheit der diagnostischen Einheit in der Probe hinweist.

3. Verfahren nach Anspruch 1, wobei das zweite fluoreszenzmarkierte Reagens einen Komplex, der das erste fluoreszenzmarkierten Reagens und ein Substrat enthält, kompetitiv zur diagnostischen Einheit bindet, und wobei die in Schritt (c) gemessene Abwesenheit oder weitgehende Abwesenheit eines Fluoreszenzsignals auf die Anwesenheit der diagnostischen Einheit in der Probe hinweist.

4. Verfahren nach Anspruch 1, wobei das zweite fluoreszenzmarkierte Reagens die diagnostische Einheit zusätzlich zu dem ersten fluoreszenzmarkierten Reagens bindet und wobei die in Schritt (c) gemessene Zunahme oder wesentliche Zunahme eines Fluoreszenzsignals auf die Anwesenheit der diagnostischen Einheit in der Probe hinweist.

5. Verfahren nach einem der vorhergehenden Ansprüche, wobei das Fluoreszenzsignal von der Fluoreszenzenergie-Donorverbindung ebenfalls gemessen wird und das Verhältnis der zwei Signale verwendet wird, um die Anwesenheit oder Abwesenheit der diagnostischen Einheit in der Probe zu bestimmen.

6. Verfahren nach einem der vorhergehenden Ansprüche, wobei eines der ersten oder zweiten fluoreszenzmarkierten Reagenzien ein Glycokonjugat einer Brucella-Spezies ist.

7. Verfahren nach einem der vorhergehenden Ansprüche, wobei das eine der ersten oder zweiten fluoreszenzmarkierten Reagenzien ein LPS-Antigen einer *Brucella*-Spezies ist und das anders ein Antikörper ist, der das Antigen bindet.

8. Verfahren nach Anspruch 7, wobei das Antigen ein O-Antigen von *Brucella* ist.

9. Verfahren nach einem der vorhergehenden Ansprüche, wobei die Fluoreszenzenergie-Donorverbindung ein Lanthanid ist.

10. Verfahren nach einem der vorhergehenden Ansprüche, wobei die Fluoreszenzenergie-Donorverbindung ein Terbium-Lanthanid-Chelat ist und die Fluoreszenzenergie-Akzeptorverbindung Fluorescein oder ein Derivat davon ist.

**11.** Verfahren nach einem der Ansprüche 1 bis 9, wobei die Fluoreszenzenergie-Donorverbindung ein Europium-Lanthanid-Chelat ist und die Fluoreszenzenergie-Akzeptorverbindung Cy5, Allophycocyanin (APC) oder ein Alexa-Fluor-Farbstoff ist.

**12.** Verfahren nach einem der vorhergehenden Ansprüche, wobei es sich bei der biologischen Probe um eine Blut-, Serum-, Plasma-, Milch-, Urin-, Schleimhautflüssigkeits-, Zerebrospinafflüssigkeks-, Stuhl- oder Gewebsbiopsieprobe handelt.

**13.** Verfahren nach einem der vorhergehenden Ansprüche, das mit mehreren Proben gleichzeitig in unterschiedlichen Reaktionsgefäßen durchgeführt wird.

**Revendications**

**1.** Procédé de détection de la présence d'un fragment diagnostique indicateur de l'exposition à un organisme infectieux de l'espèce *Brucella* dans un échantillon biologique provenant d'un être humain ou d'un animal, ledit procédé comprenant :

a) l'ajout audit échantillon d'un premier réactif marqué par fluorescence qui se lie audit fragment diagnostique, et d'un second réactif marqué par fluorescence qui se lie soit audit fragment diagnostique en plus dudit premier réactif marqué par fluorescence, soit au premier réactif marqué par fluorescence ou à un complexe comprenant le premier réactif marqué par fluorescence en compétition pour la liaison audit fragment diagnostique, dans lequel un marqueur sur l'un des premier ou second réactifs marqués par fluorescence agit comme un composé donneur d'énergie fluorescente et dans lequel l'autre desdits premier ou second réactifs marqués par fluorescence agit comme un composé accepteur d'énergie fluorescente qui est capable d'accepter l'énergie fluorescente émise par ledit composé donneur ;
b) l'excitation du composé donneur d'énergie fluorescente par exposition à une lumière ayant une longueur d'onde qui est absorbée par ledit composé donneur d'énergie fluorescente ;
c) la mesure du signal fluorescent émis par ledit composé accepteur d'énergie fluorescente suite à l'absorption par celui-ci de l'énergie fluorescente émise par le composé donneur après un laps de temps ; et
d) la corrélation des résultats à la présence ou à l'absence du fragment diagnostique dans ledit échantillon ;

dans lequel le fragment diagnostique est choisi parmi l'organisme infectieux de l'espèce *Brucella,* un antigène de l'organisme infectieux de l'espèce *Brucella,* ou un anticorps dirigé contre un antigène de l'organisme infectieux de l'espèce *Brucella,* et dans lequel les premier et second réactifs marqués par fluorescence sont des réactifs de liaison spécifiques.

**2.** Procédé selon la revendication 1, dans lequel le second réactif marqué par fluorescence se lie au premier réactif marqué par fluorescence en compétition pour ledit fragment diagnostique, et dans lequel une réduction du signal fluorescent mesuré à l'étape (c) indique la présence du fragment diagnostique dans l'échantillon.

**3.** Procédé selon la revendication 1, dans lequel le second réactif marqué par fluorescence se lie à un complexe comprenant le premier réactif marqué par fluorescence et un substrat en compétition pour ledit fragment diagnostique, et dans lequel l'absence ou l'absence substantielle du signal fluorescent mesuré à l'étape (c) indique la présence du fragment diagnostique dans l'échantillon.

**4.** Procédé selon la revendication 1, dans lequel le second réactif marqué par fluorescence se lie audit fragment diagnostique en plus dudit premier réactif marqué par fluorescence, et dans lequel l'accroissement ou l'accroissement substantiel du signal fluorescent mesuré à l'étape (c) indique la présence du fragment diagnostique dans l'échantillon.

**5.** Procédé selon l'une quelconque des revendications précédentes, dans lequel le signal fluorescent émis par le composé donneur d'énergie fluorescente est également mesuré et le rapport des deux signaux est utilisé pour déterminer la présence ou l'absence du fragment diagnostique dans l'échantillon.

**6.** Procédé selon l'une quelconque des revendications précédentes, dans lequel le premier ou le second réactif marqué par fluorescence est un glycoconjugué de l'espèce *Brucella.*

**7.** Procédé selon l'une quelconque des revendications précédentes, dans lequel le premier ou le second réactif marqué par fluorescence est un antigène LPS de l'espèce *Brucella,* et l'autre est un anticorps qui se lie audit antigène.

**8.** Procédé selon la revendication 7, dans lequel l'antigène est un antigène O de *Brucella.*

**9.** Procédé selon l'une quelconque des revendications précédentes, dans lequel le composé donneur d'énergie fluorescente est un lanthanide.

**10.** Procédé selon l'une quelconque des revendications précédentes, dans lequel le composé donneur d'énergie fluorescente est un chélate de lanthanide de type terbium, et le composé accepteur d'énergie fluorescente est la fluorescéine ou un dérivé de celle-ci.

**11.** Procédé selon l'une quelconque des revendications 1 à 9, dans lequel le composé donneur d'énergie fluorescente est un chélate de lanthanide de type europium, et le composé accepteur d'énergie fluorescence est Cy5, l'allophycocyanine (APC) ou un colorant Fluor Alexa.

**12.** Procédé selon l'une quelconque des revendications précédentes, dans lequel l'échantillon biologique est un échantillon de sang, sérum, plasma, lait, urine, muqueuse, liquide cérébro-spinal, matières fécales ou une biopsie tissulaire.

**13.** Procédé selon l'une quelconque des revendications précédentes qui est mis en oeuvre sur de multiples échantillon simultanément dans des puits de réaction séparés.

**Figure 1**

**Figure 2**

**Figure 3**

**Figure 4**

**Figure 5**

**Figure 6**

**Figure 7**

**Figure 8**

**Figure 9**

**Figure 10**

**Figure 11**

**Figure 12A**

**Figure 12B**

**Figure 13**

34

**Figure 14**

**Figure 15**

**Figure 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5622821 A
- US 5639615 A
- US 5656433 A
- US 4822733 A

- US 5627074 A
- US 20060240571 A
- US 2006234215 A

**Non-patent literature cited in the description**

- **MORRISON, L.E.** *Anal. Biochem.,* 1988, vol. 174 (1), 101
- **PORTE et al.** *Infect. Immunol.,* 2003, vol. 71 (3), 1481
- Bovine brucellosis. **NIELSEN, K. ; EWALT, D.R.** Manual of standards for diagnostic tests and vaccines. Office International Des Epizooties, 2004, 409-38

- **NEILSEN.** *Journal of Immunological Methods,* 1996, vol. 195 (1-2), 161-168
- **MINAS et al.** *Journal of Immunological Methods,* 2007, vol. 320 (1-2), 94-103
- **QIN et al.** *Clin Chem,* 2003, vol. 49, 1105-1113
- **GREISER-WILKE ; MOENNING.** *Ann Inst. Pasteur Microbiol.,* 1987, vol. 138 (5), 549-60
- OIE Manual of Diagnostic Tests and Vaccines for Terrestrial Animals. 2004